(19) Europäisches Patentamt
European Patent Office
Office européen des brevets

(11) Publication number: **0 423 618 A1**

(12) **EUROPEAN PATENT APPLICATION**

(21) Application number: **90119495.1**

(22) Date of filing: **11.10.90**

(51) Int. Cl.5: **C07H 19/052**, C07H 19/056, C07H 19/167, C07H 19/173, C07H 19/19

(30) Priority: **11.10.89 US 419988**

(43) Date of publication of application: **24.04.91 Bulletin 91/17**

(84) Designated Contracting States: **AT BE CH DE DK ES FR GB GR IT LI LU NL SE**

(71) Applicant: **MERRELL DOW PHARMACEUTICALS INC.**
**2110 East Galbraith Road**
**Cincinnati Ohio 45215(US)**

(72) Inventor: **Jarvi, Esa T.**
**3953 St. Johns Terrace**
**Cincinnati, Ohio 45236(US)**
Inventor: **Bowlin, Terry L.**
**8466 Pond Ridge Drive**
**Maineville, Ohio 45039(US)**
Inventor: **McCarthy, James R.**
**6448 Foxview Place**
**West Chester, Ohio 45069(US)**

(74) Representative: **Vossius & Partner**
**Siebertstrasse 4 P.O. Box 86 07 67**
**W-8000 München 86(DE)**

(54) **Inosine/guanosine derivatives as immunosuppressive agents.**

(57) The present invention relates to a method of effecting immunosuppression in a patient in need thereof by administering to said patient certain inosine/guanosine derivatives which are inhibitors of purine nucleoside phosphorylases.

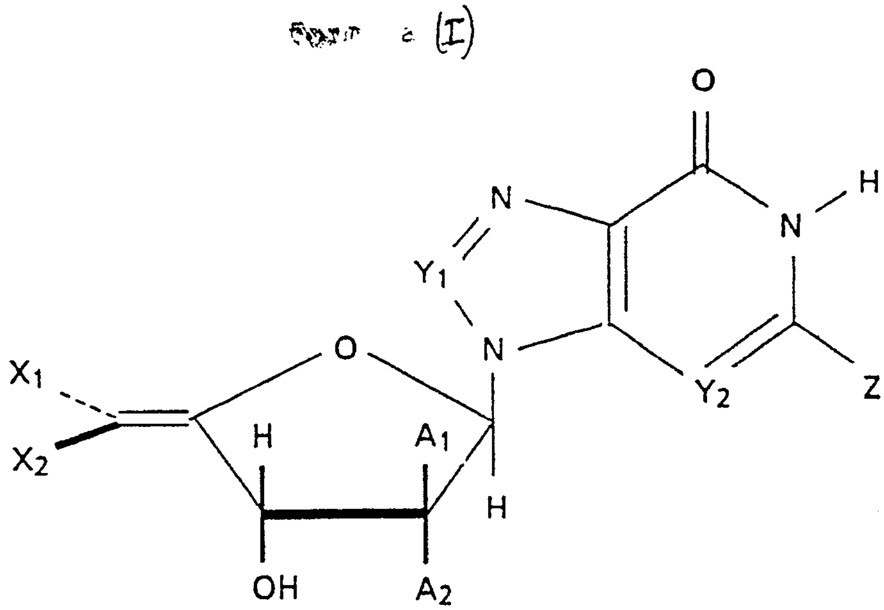
(1)

EP 0 423 618 A1

wherein
$X_1$ and $X_2$ are each independently hydrogen or halogen with the proviso that at least one of $X_1$ and $X_2$ is a halogen atom,
$A_1$ and $A_2$ are each independently hydrogen, halogen or hydroxy with the provisos that where $A_1$ is hydroxy, $A_2$ is hydrogen, and where $A_2$ is hydroxy, $A_1$ is hydrogen,
$Y_1$ is nitrogen, a CH group, a CCl group, a CBr group or a $CNH_2$ group,
$Y_2$ is nitrogen or a CH group, and
Z is hydrogen, halogen, or $NH_2$.

# INOSINE/GUANOSINE DERIVATIVES AS IMMUNOSUPPRESSIVE AGENTS

The present invention relates to certain inosine/ guanosine derivatives which are inhibitors of purine nucleoside phosphorylases and to a method of effecting immunosuppression in a patient in need thereof by administering these compounds to said patient.

Administration of these compounds provides an immunosuppressive effect. More particularly, administration of these compounds suppresses cellular immunity and inhibits lymphocyte proliferation.

Purine nucleoside phosphorylases (PNP) are enzymes present in mammalian cells and represent the primary mechanism by which animal tissues degrade the ribonucleosides and the deoxyribonucleosides of guanine and hypoxanthine (i.e., guanosine/deoxyguanosine and inosine/deoxyinosine) to the free bases and the respective pentose-1-phosphates. This reaction catalyzed by PNP is readily reversible.

Various lines of evidence have led to the conclusion that PNP inhibitors have therapeutic activity. For example, it has been suggested by Parks et al. ["Purine Nucleoside Phosphorylase and 5′-Methylthioadenosine Phosphorylase: Targets of Chemotherapy" in MOLECULE ACTIONS AND TARGETS FOR CANCER CHEMOTHERAPEUTIC AGENTS, 1981, Academic Press Inc.] that "[S]ince children with inheritable PNP deficiencies display normal humoral but defective cellular immunity, and the activity of PNP in T lymphocytes is higher than in B lymphocytes, one may predict that inhibition of PNP will have specific effects on T lymphocytes and cell-mediated immunity" (*Id.* pages 231-32). It has also been found that excessive amounts of deoxyguanosinetriphosphate (dGTP) accumulates in the tissues of PNP-deficient individuals [*Id.* page 232]. The nucleoside dGTP is a potent feed-back inhibitor of ribonucleotide reductase, an enzyme essential for the synthesis of deoxynucleotides during DNA synthesis.

Therefore, PNP inhibition would reasonably be expected to result in suppression of cell-mediated immunity and inhibition of proliferation of rapidly dividing cells. PNP inhibitors are therefore useful as immunosuppressive agents and provide effective therapy in the treatment of certain autoimmune diseases and in the prevention of rejection of transplanted organs.

The immune system is one of the primary defenses against disease bearing microbes and other foreign antigens in higher animals. An immune response is mediated by the action of specific immune cells which react to specific antigens. Potential antigens can be a variety of substances, often proteins, which are foreign to an individual's body. They are most frequently located on the outer surfaces of cells. Potential antigens can be found on pollen grains, tissue grafts, animal parasites, viruses, and bacteria.

In humans, many potential antigens never pass the body's first two defense lines and therefore never trigger the immune system. These two defense lines consist firstly of the skin, mucous membranes, tears, and stomach acid and secondly of specialized white blood cells, granulocytes and monocytes, and macrophages which destroy pathogens and other potential antigens by phagocytosis, that is, by engulfing and destroying the foreign material. These white blood cells and macrophages are called phagocytes. When pathogens or other foreign substances do pass the body's first two defense lines, the immune response begins.

There are two principal immune defense systems, humoral and cellular, both of which react to antigens. Humoral immunity is due to circulating antibodies which are found in the gamma globulin fraction of the plasma proteins. When plasma is centrifuged at high speeds its component proteins separate by weight into sections called fractions. Antibodies are usually found in the fraction whose components have a molecular weight of approximately 156,000. This particular fraction has been named the gamma globulin fraction. Humoral immunity forms a major defense against bacterial infections. Cellular immunity is partly due to lymphocyte products called lymphokines. This type of immunity is responsible for delayed allergic reactions, rejection of transplants of foreign tissue, and rejection of tumor cells. It is the major defense against infections due to viruses, fungi, and a few bacteria such as the tubercle bacillus.

Specialized white blood cells called lymphocytes are responsible for both humoral and cellular immunity. Lymphocyte precursor cells are made in the bone marrow of adult humans followed by migration to various organs or in the yolk sac of a developing fetus followed by migration into the fetus and then to various organs. In humans, some of these precursor cells migrate to the thymus, which is a two-lobed, glandular appearing structure located in the upper chest just behind the sternum, where they are transformed into T-lymphocytes, which are involved in cellular immunity. In humans, the rest of the precursor cells migrate to the spleen where they are transformed into B-lymphocytes, which are involved in humoral immunity. The T- and B-lymphocytes are structurally indistinguishable although they function differently and can be distinguished through various chemical means. The mature lymphocytes circulate in the blood and can also be found in the lymph nodes as well as the spleen and thymus.

Humoral immunity is mediated by the B-lymphocytes which have receptors for particular antigens on

their cell surfaces. They seem to be very specific and each type of B-lymphocyte reacts to only one antigen. When bacteria or viruses, for example, invade an organism, B-lymphocytes react to and combine with the antigens on the bacterial or viral surface and the lymphocyte is stimulated to divide. Its daughter cells are transformed into specialized cells called plasma cells. These cells produce and then secrete large quantities of antibodies into the general circulation. The antibodies are specific for the antigens which stimulated their production and react only with those antigens. Antibodies known as agglutinins cause several antigen containing substances to agglutinate or clump together. This keeps the substance from spreading to the tissues and allows the phagocytes to capture or the lymph nodes to filter the invading material. Other antibodies act by opening holes in bacterial cell walls, thereby killing the bacteria. These are known as lysins. Antibodies call antitoxins combine with toxins produced by bacteria and thereby neutralize them.

Once a pathogen invades the body and the immune response begins, antibodies can be made in several hours. This initial reaction is called the primary response or primary immunization. However, during that time, the pathogens have also been dividing and sometimes producing toxin, either of which results in various disease symptoms. It may take days or weeks before enough antibodies are made to eliminate all the pathogens but once they disappear, the disease symptoms disappear as well. The lymphocytes, plasma cells, and antibodies remain and circulate in the blood so that if the same pathogens enter the body a second time, the lymphocytes react immediately and start antibody production. The response of the sensitized lymphocytes is called the secondary response. The secondary response results in the production of higher levels of antibody than were produced during the primary response. So many antibodies are produced so rapidly that the microbes are unable to divide and cause disease. This type of humoral immunity is known as immediate hypersensitivity due to the fact that a previously exposed organism can respond within minutes to an antigen, as in the case of hay fever. Another example of immediate hypersensitivity would be anaphylactic shock, an extreme allergic reaction that sometimes occurs when an individual is exposed to an antigen to which he has been sensitized. At times, this humoral response to the antigen can result in death.

Humoral immunity can be both naturally and artificially induced. In the case of active natural immunity, an individual's lymphocytes continue to circulate and activate the production of antibodies after an infection. This active natural immunity lasts for many years or even a lifetime. An infant receives antibodies from the colostrum, milk secreted by the mother, the first few days after birth, which gives it immunity the first year of its life. This is known as passive natural immunity since the infant is not involved in the actual production of the antibodies. Active artificial immunity is induced by injecting dead or weakened microbes into an individual. Their surface antigens can still trigger lymphocyte production of antibodies but these microbes do not cause the disease symptoms that their more virulent forms do. When the individual is later exposed to the virulent microbe, he is already sensitized and immediately responds with a massive production of antibodies. Active artificial immunity may last many years or permanently with booster shots. There is also a form of passive artificial immunity which provides protection for about one month. This temporary immunity is brought about by injecting antibodies obtained from another person or animal into an individual. It is usually only used in crisis situations and epidemics. Because the lymphocytes are bypassed, they neither make antibodies nor "remember" the antigen, which accounts for the temporary effect of this method.

In cellular immunity, as contrasted to humoral-immunity, circulating antibodies are not detectable. The T-lymphocytes which mediate this type of immunity are activated when they encounter antigens on cells from another individual, as in the case of transplants, tumors, or viruses. Like B-lymphocytes, T-lymphocytes are specific and each type reacts with only one antigen. The lymphocytes enlarge, divide, and produce lymphokines which participate in the attack on the foreign antigen. They also stimulate the phagocytic activity of macrophages. Although immunological memory exists as with humoral immunity, the response is much slower. It may take as long as ten or twelve hours to develop a response in a previously sensitized individual and cellular immunity is therefore known as delayed hypersensitivity. The allergic reaction to poison ivy, oak, and sumac, the red splotch seen in a positive tuberculin skin test, and rejection of transplant tissue are all cellular immune responses.

Immunomodulating agents activate or inhibit the process of lymphocyte proliferation. Normal lymphocyte proliferation is due to various interactions between antigens, macrophages, T-and B-lymphocytes as well as certain chemicals. For example, the presence of a particular antigen activates a particular T-or B-lymphocyte. Additionally, certain B-lymphocytes can be activated by active T-lymphocytes while others are independent of the T-lymphocytes and are activated only by antigens. Activated T-lymphocytes can cause macrophages to produce a molecule known as interleukin 1(IL-1) which in turn activates both T- and B-lymphocytes. Activated T-lymphocytes can also produce a molecule known as interleukin 2 (IL-2) which

further induces T-lymphocyte activation. Chemicals, called mitogens can trigger DNA synthesis and mitosis, which are signs of activity in T- or B-lymphocytes. Some mitogens affect only one type of lymphocyte while others affect many types. Immunomodulating agents of various kinds and in varying amounts affect the complex interactions between the components of the immune system.

Although the immune system is a major defense against substances which can cause disease, it cannot distinguish between helpful and harmful foreign substances and destroys both. It would be useful in many instances to have a means of regulating the immune system without harming the individual.

There are times when the individual's immunological response causes more damage or discomfort than the invading microbes or foreign material, as in the case of allergic reactions. Suppression of the immune response in these cases would be desirable.

Occasionally, the immunological mechanisms become sensitized to some part of the individual's own body causing interference with or even destruction of that part. The ability to distinguish between "self" and "not self" is impaired and the body begins to destroy itself. This can result in an autoimmune disease. Some examples of these autoimmune diseases in man are rheumatoid arthritis, certain hemolytic anemias, rheumatic fever, thyroiditis, ulceractive colitis, myestheniagravis, glomerulonephritis, allergic encephalomyelitis, continuing nerve and liver destruction which sometimes follows viral hepatitis, multiple sclerosis and systemic lupus erythematosus. Some forms of autoimmunity come about as the result of trauma to an area usually not exposed to lymphocytes such as neural tissue or the lens of the eye. When the tissues in these areas become exposed to lymphocytes, their surface proteins can act as antigens and trigger the production of antibodies and cellular immune responses which then begin to destroy those tissues. Other autoimmune diseases develop after exposure of the individual to antigens which are antigenically similar to, that is cross-react with, the individual's own tissue. Rheumatic fever is an example of this type of disease in which the antigen of the streptococcal bacterium which causes rheumatic fever is cross-reactive with parts of the human heart. The antibodies cannot differentiate between the bacterial antigens and the heart muscle antigens and cells with either of those antigens can be destroyed. Suppression of the immune system in these autoimmune diseases would be useful in minimizing or eliminating the effects of the disease.

Circulating antibodies and cellular immune responses play a role in the rejection of transplanted tissues and organs. Unless the donor is the identical twin of the recipient or is the individual himself, the recipient's lymphocytes recognize the transplant as "not self" and immediately respond to destroy it. The exceptions to this situation are transplants to nonvascularized areas (privileged sites), such as the cornea of the eye, where lymphocytes do not circulate and therefore are not sensitized and do not prompt an immune response. It is currently difficult to suppress the immune reaction to prevent rejection of the transplant without severely damaging the patient in other ways. The patient must also be given massive doses of antibiotics because his own defenses against infection have been suppressed. Suppression of the immune system would thus be useful in preventing such rejection of transplant tissues.

The present invention provides novel inosine/guanosine derivatives of formula (1) and a method of effecting immunosuppression in a patient in need thereof comprising administering to said patient a therapeutically effective immunosuppressive amount of a compound of formula (1)

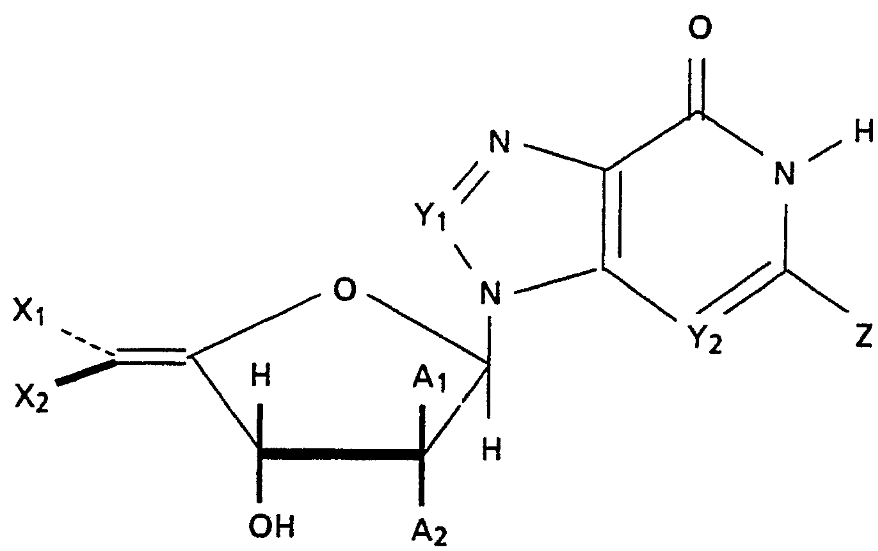

(1)

wherein
$X_1$ and $X_2$ are each independently hydrogen or halogen with the proviso that at least one of $X_1$ and $X_2$ is a halogen atom,
$A_1$ and $A_2$ are each independently hydrogen, halogen or hydroxy with the provisos that where $A_1$ is hydroxy, $A_2$ is hydrogen, and where $A_2$ is hydroxy, $A_1$ is hydrogen,
$Y_1$ is nitrogen, a CH group, a CCl group, a CBr group or a $CNH_2$ group,
$Y_2$ is nitrogen or a CH group, and
Z is hydrogen, halogen, or $NH_2$.

More particularly, the present invention provides a method for suppressing cellular immunity and for inhibiting lymphocyte proliferation in patients in need thereof comprising administering a therapeutically effective immunosuppressive amount of a compound of the formula (1).

As used herein, the terms "halogen" or "halo-" refer to a monovalent fluorine, chlorine, bromine or iodine atom. Of course, it will be appreciated by those skilled in the art that the inosine/guanosine derivatives of the present invention can exist in the keto or enol form. For purposes of convenience, the structures of these inosine/guanosine derivatives are presented in the keto form only. The present invention is not limited to any one form of the inosine/guanosine derivatives and both the keto and enol forms are included within the scope of the invention. It will further be appreciated that the inosine/guanosine derivatives of the present invention can exist in various stereoisomeric forms. The present invention is not limited to any particular stereoisomeric forms and includes all such stereoisomers individually and as racemic mixtures.

The inosine/guanosine derivatives of the present invention can be prepared by utilizing procedures and techniques well known and appreciated by one of ordinary skill in the art.

A general synthetic procedure for making inosine/guanosine derivatives of the formula (1), wherein Z is other than $NH_2$, is set forth in Scheme A. In this scheme the compounds of formula (1) are made from the corresponding 4′-vinylhalo-adenosine derivatives. The term $Z_1$ refers to hydrogen or halogen and all other substituents, unless otherwise indicated, are as previously defined.

Scheme A

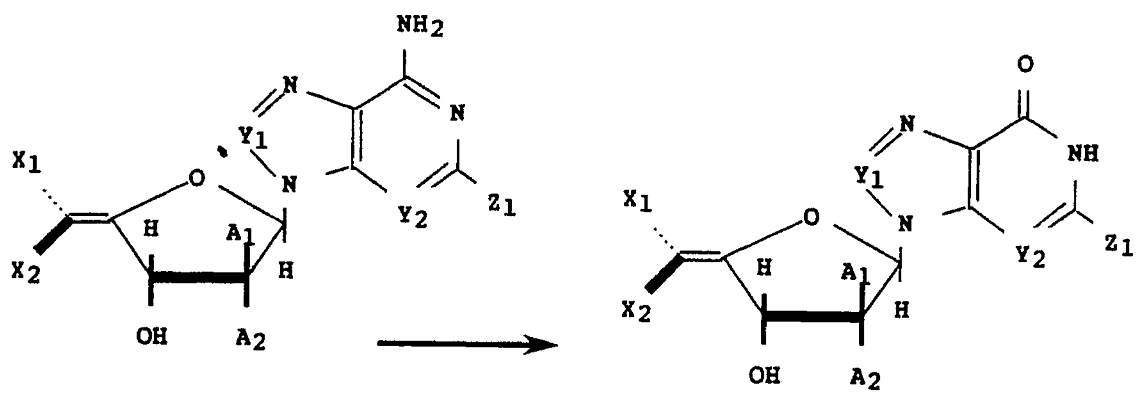

(2)

(3)

In this reaction, compounds of the formula (1) are made by subjecting a corresponding 4′-vinylhalo-adenosine derivative to an acidic oxidative deamination.

In Scheme A, the 4′-vinylhalo-adenosine derivative (2) is subjected to an oxidative deamination by means of an acidic oxidative agent to yield the corresponding 4′-vinylhaloinosine/guanosine derivative (3). The preferred acidic oxidative agent for this reaction is nitrous acid. Where nitrous acid is utilized, the 4′-vinylhalo-adenosine derivative (2) can be dissolved in an organic acid such as acetic acid and sodium nitrite can be added to form the nitrous acid *in situ*. The reaction can generally be carried out at ambient temperature and is completed within several hours.

The following examples present typical syntheses as described by Scheme A. These examples are understood to be illustrative only and are not intended to limit the scope of the present invention in any way. As used herein the following abbreviated terms have the following meanings: mg refers to milligrams, mmol refers to millimoles, mL refers to milliliters, v refers to volume.

## EXAMPLE 1

### (Z)-5′-Fluoro-4′,5′-didehydro-5′-deoxyinosine

Dissolve (Z)-5′-fluoro-4′,5′-didehydro-5′-deoxyadenosine (267 mg, 1 mmol) in glacial acetic acid (10 mL) with stirring. To this reaction mixture add a solution of sodium nitrite (276 mg, 4 mmol) in water (2 mL). Stir the mixture for 5 hours at ambient temperature. Evaporate the solvent to dryness *in vacuo* and triturate the resulting solid with boiling acetone. Evaporate the acetone extracts to dryness. Recrystallize the title compound from ethanol/water.

## EXAMPLE 2

### (Z)-5′-Chloro-4′,5′-didehydro-5′-deoxyinosine

Dissolve (Z)-5′-chloro-4′,5′-didehydro-5′-deoxyadenosine (284 mg, 1 mmol) in glacial acetic acid (10 mL) with stirring. To this reaction mixture add a solution of sodium nitrite (276 mg, 4 mmol) in water (2 mL). Stir the mixture for 5 hours at ambient temperature. Evaporate the solvent to dryness *in vacuo* and triturate the resulting solid with boiling acetone. Evaporate the acetone extracts to dryness. Recrystallize the title compound from ethanol/water.

The 4′-vinylhalo-adenosine derivatives (2) which are useful as starting materials in the reaction described in Scheme A, can be prepared by utilizing procedures and techniques which are well known and appreciated by those of ordinary skill in the art.

For example, the 4′-vinylhalo-adenosine derivatives, wherein one of $X_1$ and $X_2$ is hydrogen, can be prepared by a general synthetic procedure as set forth in Scheme B wherein the term "halogen" or "$X_{Hal}$" refers to a fluorine, chlorine, bromine, or iodine atom and the term "nitrogen" refers to a trivalent nitrogen atom attached to two radicals and all other substituents, unless otherwise indicated, are as previously defined.

SCHEME B

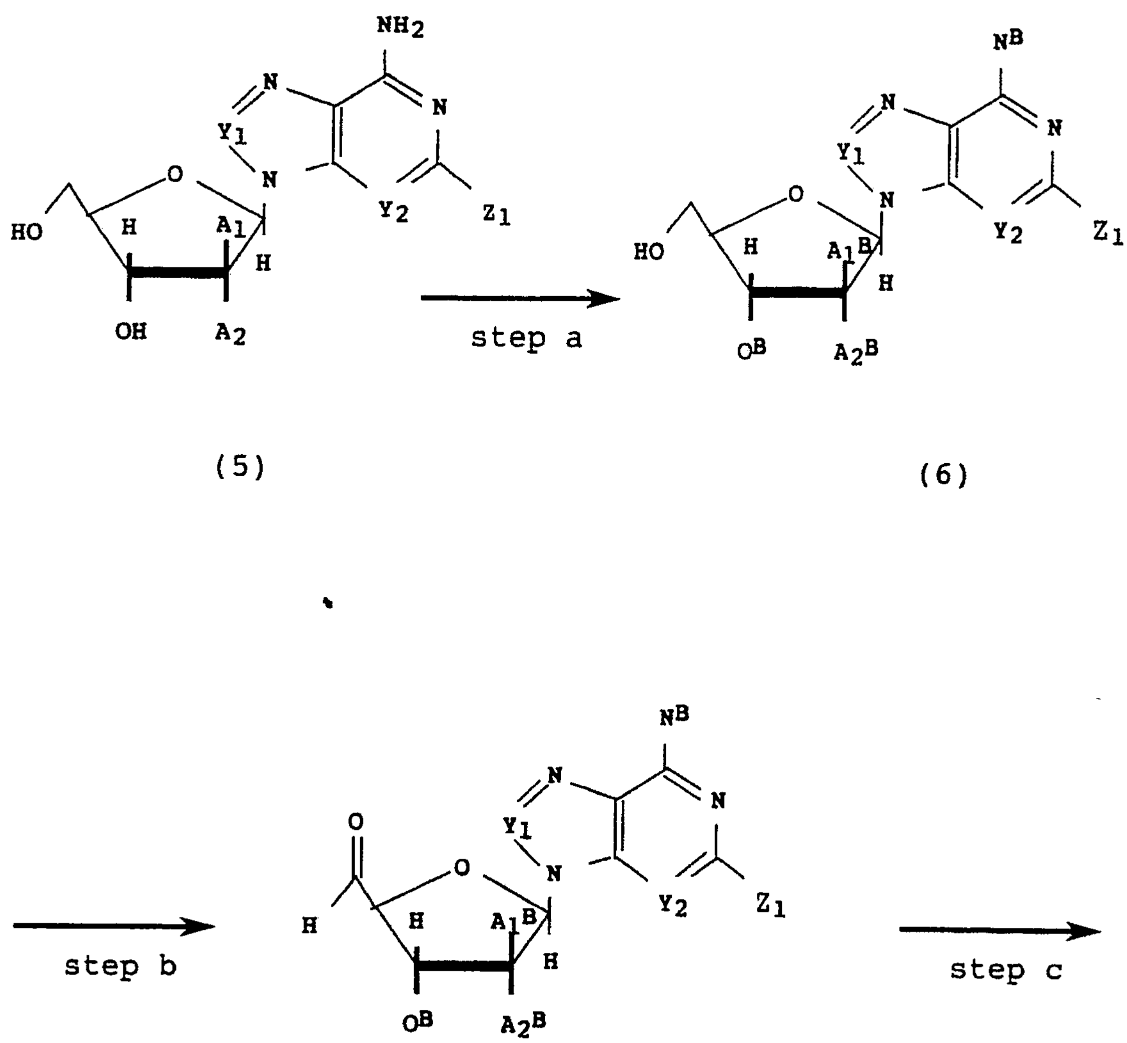


(5) (6)

(7)

Scheme B (cont'd)

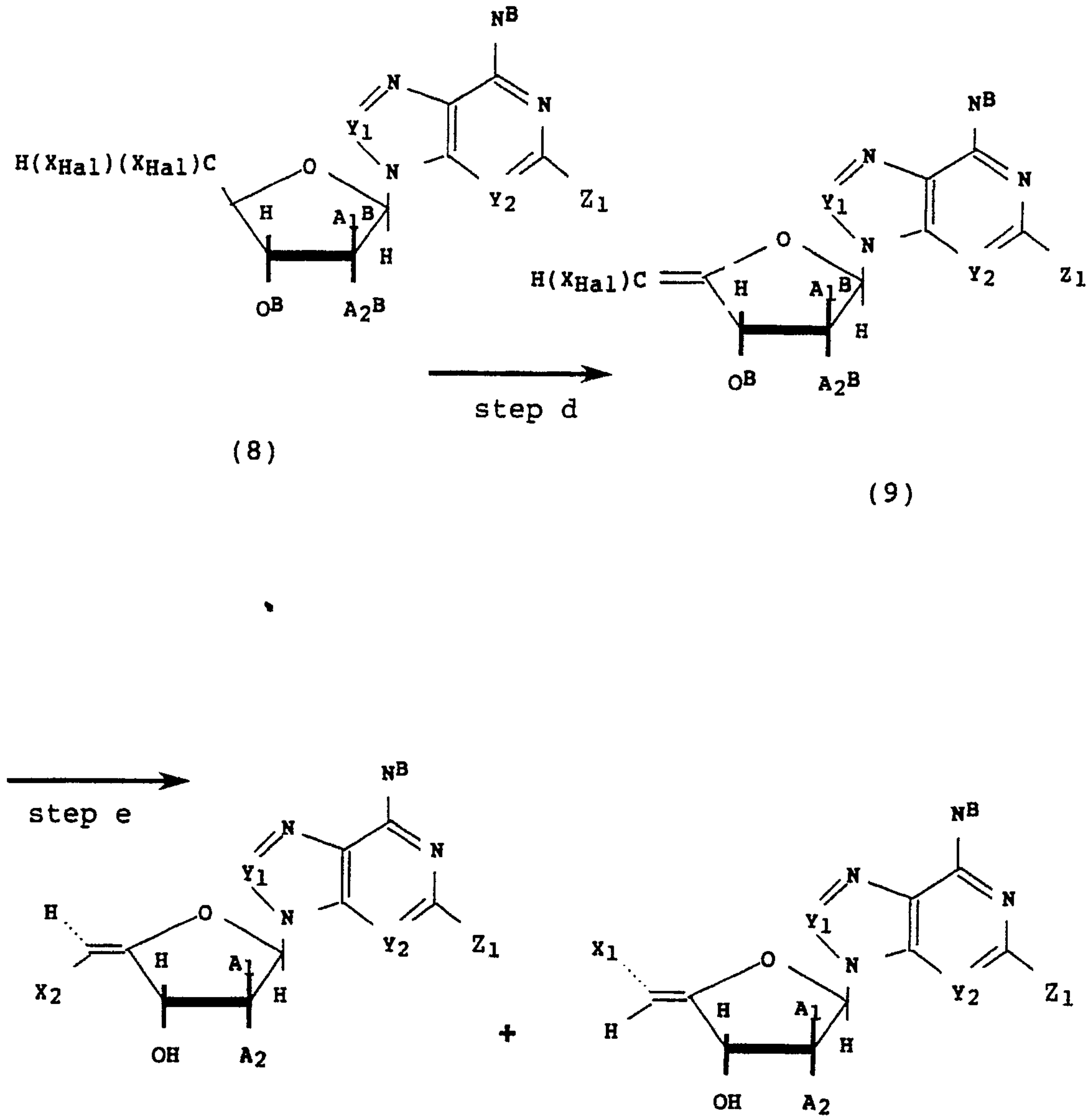


(8)

(9)

(10) + (11)

Scheme B (cont'd)

(10)

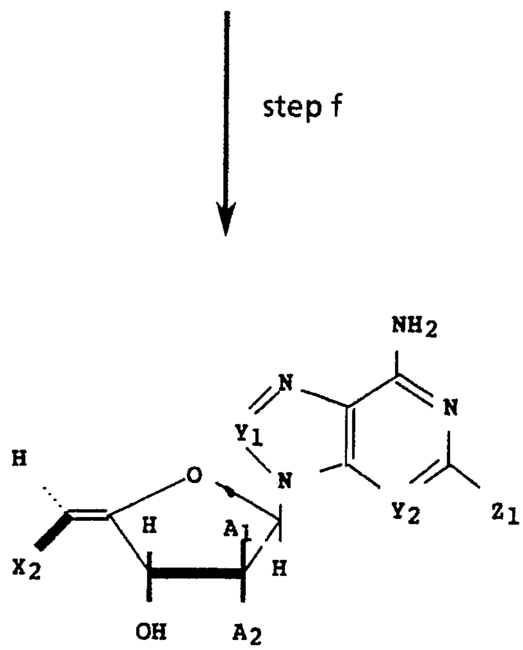


(12)

(11)

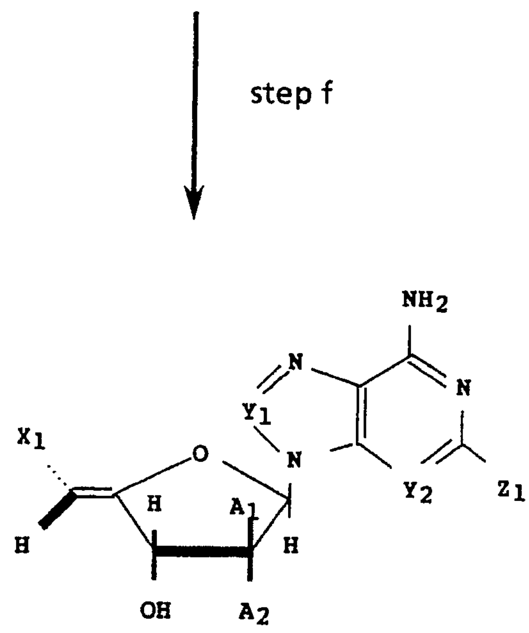


(13)

Basically, in step a, reactive hydroxy or amino groups other than the 5'-hydroxy group are blocked with standard blocking agents well known in the art. These blocking groups can be conventional amino protecting groups for the 6-amino, and conventional hydroxy protecting groups for the 3'-hydroxy and for $A_1$ and $A_2$ (wherein $A_1$ or $A_2$ are OH). $O^B$, $A_1{}^B$, $A_2{}^B$ and $N^B$ in Scheme B represent the 3'-hydroxy, $A_1$, $A_2$ and the 6-amino groups as herein defined blocked with a blocking group where appropriate.

The selection and utilization of particular blocking groups are well known to one of ordinary skill in the art. In general, blocking groups should be selected which adequately protect the amino or hydroxy groups in question during subsequent synthetic steps and which are readily removable under conditions which will not cause degradation of the desired product.

Examples of suitable hydroxy protecting groups are $C_1$-$C_6$ alkyl, tetrahydropyranyl, methoxymethyl, methoxyethoxymethyl, t-butyl, benzoyl, and triphenylmethyl. The term $C_1$-$C_6$ alkyl refers to a saturated hydrocarbyl radical of one to six carbon atoms of straight, branched, or cyclic configuration. The preferred blocking group for the 3'-hydroxy and for $A_2$ (wherein $A_2$ is hydroxy) is 2',3'-0-isopropylidene. The preferred blocking group for $A_1$ (wherein $A_1$ is hydroxy) and for the 3'-hydroxy (wherein $A_2$ is not hydroxy) is benzoyl. The 2',3'-0-isopropylidene derivative can be formed by reacting the unblocked compound with acetone. The benzoylated derivative can be formed by reacting the unblocked compound with benzoyl chloride in the presence of pyridine.

Examples of suitable amino protecting groups are benzoyl, formyl, acetyl, trifluoroacetyl, phthalyl, tosyl, benzenesulfonyl, benzyloxycarbonyl, substituted-benzyloxycarbonyl (e.g., p-chloro,p-bromo, p-nitro, p-methoxy, o-chloro, 2,4-dichloro, and 2,6-dichloro derivatives), t-butyloxycarbonyl (Boc), t-amyloxycarbonyl, isopropyloxycarbonyl, 2-(p-biphenyl)-isopropyloxycarbonyl, allyloxycarbonyl, cyclopentyloxycarbonyl, cyclohexyloxycarbonyl, adamantyloxycarbonyl, phenylthiocarbonyl, and triphenylmethyl. Preferred amino protected compounds include the di-benzoyl derivative, made by reacting the unblocked compound with benzoyl chloride, and the acetyl derivative, made by reacting the unblocked compound with acetic

anhydride. It is particularly preferred to protect the 6-amino group as the $N^6$,$N^6$-di-benzoyl derivative.

In step b, the appropriately blocked 5′-hydroxy derivative (6) is oxidized to the corresponding aldehyde (7). The preferred oxidizing reagent is dicyclohexylcarbodiimide, methyl phosphonic or dichloroacetic acid and dimethylsulfoxide.

The aldehyde (7) can optionally be derivatized so as to improve the handling characteristics of the compound or to facilitate purification thereof by means of procedures and techniques well known and appreciated in the art. For example, the 5′,5′-(N,N′-diphenylethylenediamino) derivative can be prepared by the method of Ranganathan et al. (J. Org. Chem., 39 , 290 (1974)].

In step c, the 5′,5′-di-halo derivative (8) is formed by reacting the corresponding aldehyde (7) with a diethylaminosulfur trihalide or similar halo-substituting reagent. Diethylaminosulfur trihalide is preferred.

In step d, the 5′-di-halo derivative (8) is dehydrohalogenated to form the unsaturated (i.e., "(H)($X_{Hal}$)C") derivative (9). The preferred reagent to effect the dehydrohalogenation is potassium t-butoxide in the presence of dimethylsulfoxide.

In step e, the hydroxy protecting groups are removed according to conventional procedures and techniques well known and appreciated in the art. For example, a 2′,3′-0- isopropylidene blocking group can be removed by reacting (9) with aqueous trifluoroacetic acid. The (Z) and (E) isomers, i.e., (10) and (11), respectively, can conveniently be isolated at this stage of the synthesis by the utilization of conventional isolation techniques as are well known and appreciated in the art. Alternatively, the (Z) and (E) isomers can be isolated after deblocking the amino-protecting groups as described below for steps f and g.

In step f, the amino-protecting groups of the (Z) and (E) isomers, i.e., (10) and (11) respectively, are removed utilizing procedures and techniques well known and appreciated in the art. For example, the benzoyl amino blocking groups can be removed by an aminolysis reaction with ammonia.

Starting materials for use in the general synthetic procedure outlined in Scheme B are readily available to one of ordinary skill in the art. For example, certain starting materials for various compounds of formula (1) are listed in Table 1.

TABLE 1

| Examples of Starting Materials for Scheme B | | | | | |
|---|---|---|---|---|---|
| Compound of formula (1) wherein | | | | | |
| $A_1$ | $A_2$ | $Y_1$ | $Y_2$ | Z | Source of Starting Material |
| H | OH | CH | CH | H | J. Med. Chem 25, 626(1982) |
| OH | H | CH | N | H | Het. Chem 14, 195(1977) |
| H | H | CH | N | H | 2′-Deoxyadenosine (commercially available) |
| OH | H | CH | N | F | JACS 86 , 1242 (1964) |

Additional starting materials can be prepared by the use of methods analogous to those described in Table 1 as well as other conventional methods as are well known and appreciated in the art.

The following example presents a typical synthesis as described by Scheme B. This example is understood to be illustrative only and is not intended to limit the scope of the present invention in any way.

## EXAMPLE 3

### (Z) and (E)-4′,5′-Didehydro-5′-deoxy-5′-fluoroadenosine

Step a: $N^6$-benzoyl-2′,3′-0-isopropylidene-adenosine.

Convert adenosine to its 2′,3′-acetonide followed by benzoylation to the $N^6$-benzoyl derivative according to the procedure of Smrt et al. [Coll. Czech. Chem. Comm. 29 , 224 (1964)].

Step b: N6,N6-Bis benzoyl-5-deoxy-2′,3′-0-isopropylidene-5′-,5′-(N,N′-diphenylethylenediamino) adenosine.

Convert N6-benzoyl-2′,3′-0-isopropylidene adenosine to N6-benzoyl-5′-deoxy-2′ ,3′-0-isopropylidene-5′ ,5′-(N,N′-diphenylethylenediamino)adenosine according to the procedure of Ranganathan et al. [J. Org. Chem. 39 , 290 (1974)]. To 2.96 g of this product in 10 ml of pyridine, cooled in an ice bath, add 1.15 ml (9.9 mmol) of benzoyl chloride. Stir the mixture overnight at room temperature and pour into ice water. Extract the product into 100 ml of chloroform and dry with magnesium sulfate. Evaporate the solution on a rotary evaporator and add toluene. Repeat the evaporation *in vacuo*, and collect 4.07 g of a yellow foam. Percolate the product through a 40 mm X 10 cm flash silica gel column with 4% ethyl acetate/96% dichloromethane. Combine and evaporate the appropriate fractions and collect a yellow oil. Dissolve the oil in ethanol and evaporate three times to yield a solid. Triturate the solid with 50 ml of ethanol and filter. Dry the solid *in vacuo* to give 2.67 g of the title compound [mp 135-138 degrees Celsius (° C)].

NMR ($CDCl_3$, 90 MHz): δ1.30 (3H, 5) 1.50 (3H, 5), 3.3-3.7 (4H, m), 4.55 (1H, m), 5.1 (2H, d, J = 2), 5.65 (1H, d, J = 2), 6.1 (1H, S), 6.3-7.8 21H, M), 8.40 (1H, S).

Step b continued: N6,N6-Bis benzoyl-2′,3′-0-isopropylidene adenosine-5′-aldehyde.

To 2.64 g (3.73 mmol) of N6,N6-Bis-benzoyl-5′-deoxy-2′ ,3′-0-isopropylidene-5′ ,5′-(N,N′-diphenylethylenediamino)adenosine in 370 ml of dichloromethane at 0° C add a solution of 1.56 g (8.2 mmol) p-toluenesulfonic acid monohydrate in 180 ml of acetone. Stir the mixture for 1.5 hours and filter. Evaporate the filtrate on a rotary evaporator and partition the residue between 200 ml of dichloromethane and water. Dry the dichloromethane solution with magnesium sulfate and evaporate to a foam. Dissolve 2.10 g of the foam in 200 ml of benzene and reflux in a Dean-Stark apparatus for one hour. Evaporate the solvent to give 2.06 g of the title compound. (NMR Spectrum reveals more than 80% of the product as aldehyde.)

NMR ($CDCl_3$, 90 MHz): δ 1.40 (3H, S) 1.70 (3H, S), 4.65 (1H, S), 5.3 (1H, d, J = 7), 5.45 (1H, broad d, J = 7), 6.2 (1H, S), 7.2-7.8 (10H, m), 8.10 (1H, S), 8.45 (major) and 8.55 (1H together, two S). 9.3 (1H, S, CHO).

Step c: N6,N6-Bis-benzoyl-5′-deoxy-5′,5′-difluoro-2′,3′-0-isopropylideneadenosine.

Chromatograph 6.5 g of N6,N6-bis-benzoyl-2′,3′-0-isopropylideneadenosine-5′-aldehyde on a 40 mm x 7 cm flash silica gel column with 15% ethyl acetate/85% dichloromethane solvent. Combine and evaporate all fractions with UV -active material on Thin Layer Chromatography (TLC) to give 5.2 g of a foam. Reflux the foam in 200 ml of benzene for 2 hours and then evaporate and dry *in vacuo* to give 4.65 g of purified N6,N6-bis-benzoyl-2′3′-0-isopropyiideneadenosine-5′-aldehyde. Dissolve 3.90 g of the 5′-aldehyde in 25 ml of dichloromethane (distilled from calcium hydride) and to this solution add 3.2 ml (3 equivalents) of diethylaminosulfur trifluoride. Stir the mixture for 6 hours. Dilute the mixture with chloroform and pour into 50 ml of stirred saturated aqueous sodium bicarbonate. Extract the product into 400 ml of chloroform and dry with $MgSO_4$. Evaporate the solvent to give 3.60 g of a foam. Percolate the product through a 40 mm x 12 cm silica gel flash column with 4% ethyl acetate/96% dichloromethane solvent. Isolate the title compound (738 mg) by TLC ($R_f$ 0.6 with 10% ethyl acetate/90% dichloromethane as solvent).

NMR ($CDCl_3$, 300 MHz): δ 1.42 (3H, S) 1.65 (3H, 5) 4.42-4.53 (1H, three m), 5.27 (1H, dd, J = 2.7, 5.9), 5.39 (1H, dd, J = 1.7, 6.0), 5.96 (1H, td, J = 55, 4.5), 7.34- 7.52 (6H, m), 7.85 (4H, d J = 7.2), 8.15 (1H, 5), 8.67 (1H, S).

19F-NMR ($CDCl_3$, 282 MHz, ppm from external $CFCl_3$)- 54.87 (ddd, J = 12.4, 55.2, 299.0)
- 50.71 (ddd, J = 10, 55.2, 299.1)

MS (FAB - XENON) M + 1 = 536

Anal: Calc'd for $C_{27}H_{23}F_2N_5O_5$. C 60.56, H 4.33

Found: C 60.26, H 4.44

Step d: N6 Benzoyl-4′,5′-didehydro-2′,3′-0-isopropylidene-5′-deoxy-5′-fluoroadenosine

To 401 mg (0.75 mmol) of crushed N6,N6-Bis-benzoyl-5′-deoxy-5′,5′-difluoro-2′,3′-0-isopropylideneadenosine and 335 mg (4 equivalents) of potassium t-butoxide under nitrogen add 2 ml of

dimethylsulfoxide (distilled from calcium hydride). Stir the mixture under nitrogen for 21 hours. Quench with 4 ml of saturated ammonium chloride and extract with ethyl acetate to yield 274 mg of yellow oil. Percolate the oil through a 20 mm x 15 cm flash column with 30% ethyl acetate/70% dichloromethane. Combine fractions that have two spots close together at Rf = 0.55 (TLC with ethyl acetate as solvent). Evaporate these fractions to yield 183 mg of the title compound containing two isomers in a 2:1 ratio.

NMR ($CDCl_3$, 300 MHz): δ 1.34 and 1.37 (minor) 3H together two S.), 1.49 (3H, s), 5.35-5.38 (1H, m), 5.56 and 5.90 (1H together; d, J=4 and m, resp.), 6.23 (broad s, minor) and 6.25 (1H together), 6.43 (d, J=74, major) and 6.81 (d, J=77; 1H together), 7.39-7.98 (6H, m), 8.646 (major) and 8.653 (minor; two s, 1H together), 9.05 (1H, broad, NH)

NMR $^{19}F$, 282 MHz, ppm from external $CFCl_3$): δ- 158.94 (d, J=74 major), 174.4 (d, J=77, minor) MS: (CI) M+1 = 412.

Step e: $N^6$-Benzoyl-4′,5′-didehydro-5′-deoxy-5′-fluoro adenosine

Dissolve 178 mg of $N^6$-benzoyl-4′,5′-didehydro-2′,3′-0-isopropylidene-5′-deoxy-5′-fluoroadenosine (2:1 mixture of isomers) in 2 ml of cold trifluoroacetic acid-water (4:1). Stir the mixture at room temperature for 50 minutes and then evaporate on a rotary evaporator. Chromatograph the residue on a 20 mm x 14 cm flash silica gel column with ethyl acetate as the solvent. Combine fractions to give 3 mg of the higher $R_f$ isomer (minor isomer), 58 mg of a mixture of isomers and 83 mg of the lower $R_f$ isomer (major isomer) of the title compound.

NMR ($CD_3OD$, higher $R_f$ isomer, 90 MHz): δ 5.1 (2H, m), 6.35 (1H, d, J=6), (1H, D, J=74), 7.5-8.2 (5H, m), 8.63 (1H, s), 8.72 (1H, 5).

NMR ($CD_3OD$, major lower $R_f$ isomer, 90 MHz): δ 5.00-5.10 (2H, m), 6.37 (1H, d, J=7), 6.48 (1H, a, J=75), 7.54-8.19 (5H, m), 8.53 (1H, s), 8.62 (1H, s).

Step f: (Z)-4′,5′-didehydro-5′-deoxy-5′-fluoroadenosine.

Dissolve 83 mg of $N^6$-benzoyl-4′,5′-didehydro-5′-deoxy-5′-fluoroadenosine (lower $R_f$ isomer above) in absolute ethanol, evaporate and redissolve in 6 ml of ethanol. Bubble anhydrous ammonia through the ice cooled solution in a 20 mm x 12 cm Carius tube. Seal the tube and remove the ice bath. After 14 hours at room temperature, open the tube and evaporate the solution to give 87 mg of crude product. Triturate in 1 ml of methanol and filter off the solid. Dry the product *in vacuo* to give 20 mg of the title compound (a white powder, softens at 100-110° C and melts at 225-230° C).

NMR ($CD_3OD$, 300 MHz): δ 5.02-5.05 (2H, m), 6.28 (1H, d, J=F), 6.56 (1H, d, J=7.52), 8.21 (1H, s), 8.33 (1H, s).

$^{19}F$-NMR (282 MHz, ppm from external $CFCl_3$):

-166.76 (d, J=75.2)

MS: (FAB-XENON) M + 1 = 268

Step f: 4′,5′-didehydro-5′-deoxy-5′-fluoroadenosine,with E-isomer as major component.

Dissolve 58 mg of $N^6$-benzoyl-4′,5′-didehydro-5′-deoxy-5′-fluoroadenosine (a mixture with the higher $R_f$ isomer being the major isomer) in 5 ml of absolute ethanol, and bubble ammonia through the ice cooled solution in a 20 mm x 12 cm Carius tube for 3 three minutes. Seal the tube and remove the ice bath. After 15 hours at room temperature, open the tube and evaporate the solution. Dissolve the residue in 2 ml of methanol and chromatograph on a 20 mm x 12 cm silica gel flash column. Eluted with ethyl acetate, followed by 10% methanol/90% ethyl acetate. Combine and evaporate fractions containing material at $R_f$ 0.23 (10% methanol/90% ethyl acetate) to yield 30 mg of product. Triturated in 12 mg of methanol and filter off the solid. Dry the product *in vacuo* to yield 16 mg of the title compound (an off-white powder). NMR indicates a 4:1 mixture of E-isomer to Z-isomer.

′H-NMR (E-isomer $CD_3OD$ 300 MHz): δ5.03-5.07 (2H, m) 6.21 (1H, d, J=6.3), 7.02 (1H, d, J=78.6), 8.20 (1H, s), 8.32 (1H, s).

$^{19}F$-NMR (E-isomer, $CD_3OD$, 282 MHz, ppm from ext. $CFCl_3$): -182.30 (d, J=78.5).

MS: (CI) mH+ =268.

The following specific compounds can be made by procedures analogous to those described above in

Example 3:
(E) and (Z)-5′-Bromo-4′,5′-didehydro-5′-deoxyadenosine
(E) and (Z)-5′-chloro-4′,5′-didehydro-5′-deoxyadenosine
(E) and (Z)-5′-Bromo-4′,5′-didehydro-2′,5′-dideoxyadenosine
(E) and (Z)-5′-chloro-4,5′-didehydro-2′,5′-dideoxyadenosine
(E) and (Z)-5′-Fluoro-4′,5′didehydro-2′,5′-dideoxyadenosine

These adenosine derivatives can then be converted to the appropriate inosine/guanosine derivatives of formula (1) by the utilization of the procedure as described in Scheme A to yield the following specific compounds:
(E) and (Z)-5′-Bromo-4′,5′-didehydro-5′-deoxyinosine
(E) and (Z)-5′-Chloro-4′,5′-didehydro-5′-deoxyinosine
(E) and (Z)-5′-Fluoro-4′,5′-didehydro-5′-deoxyinosine
(E) and (Z)-5′-Bromo-4′,5′-didehydro-2′,5′-dideoxyinosine
(E) and (Z)-5′-Chloro-4′,5′-didehydro-2′,5′-dideoxyinosine
(E) and (Z)-5′-Fluoro-4′,5′-didehydro-2′,5′-dideoxyinosine

The 4′-vinylhalo-inosine/guanosine derivatives of the formula (1), wherein one of $X_1$ and $X_2$ is hydrogen and the other is halogen, can alternately be prepared by a procedure as described in Scheme C, wherein all terms are as previously defined and the term "4-MeO-$\phi$-" refers to a 4-methoxyphenyl group.

SCHEME C

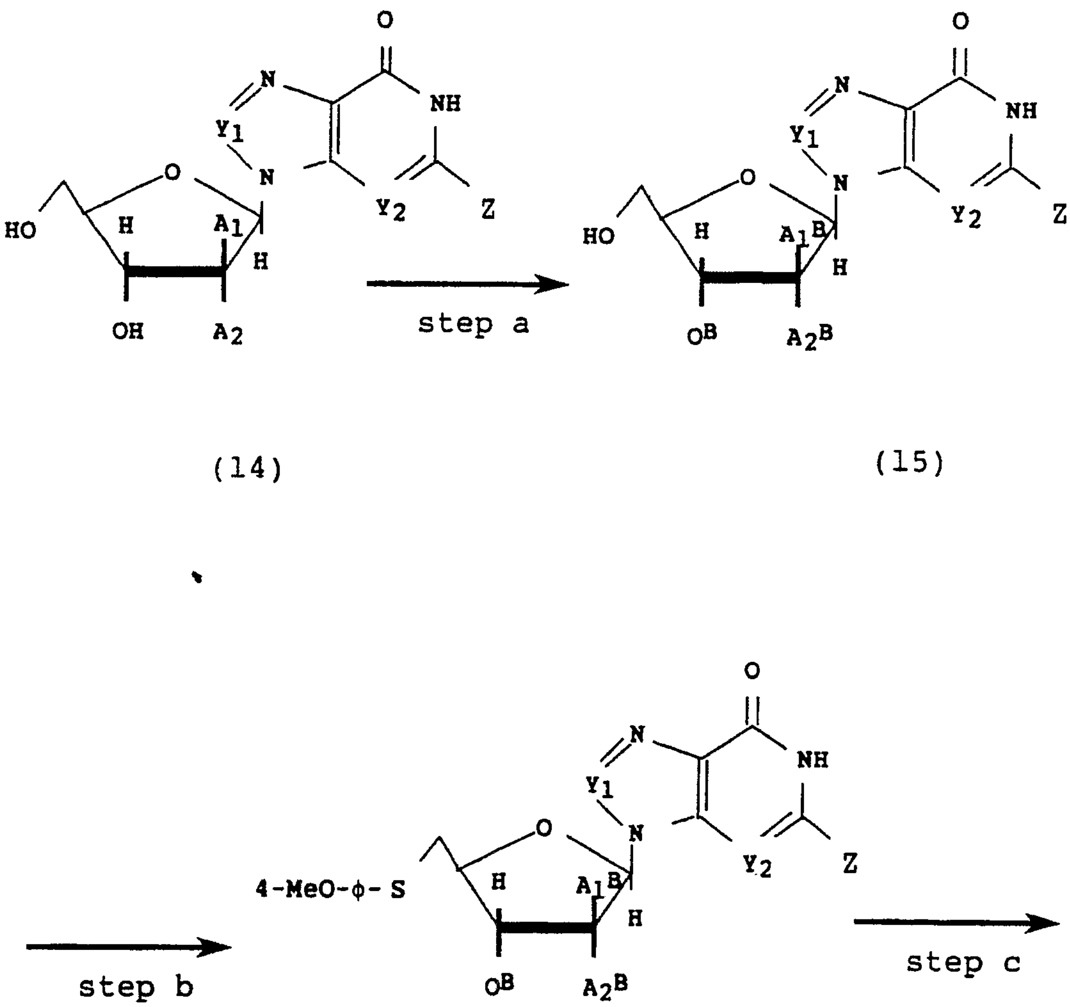


(14)

(15)

(16)

Scheme C (cont'd)

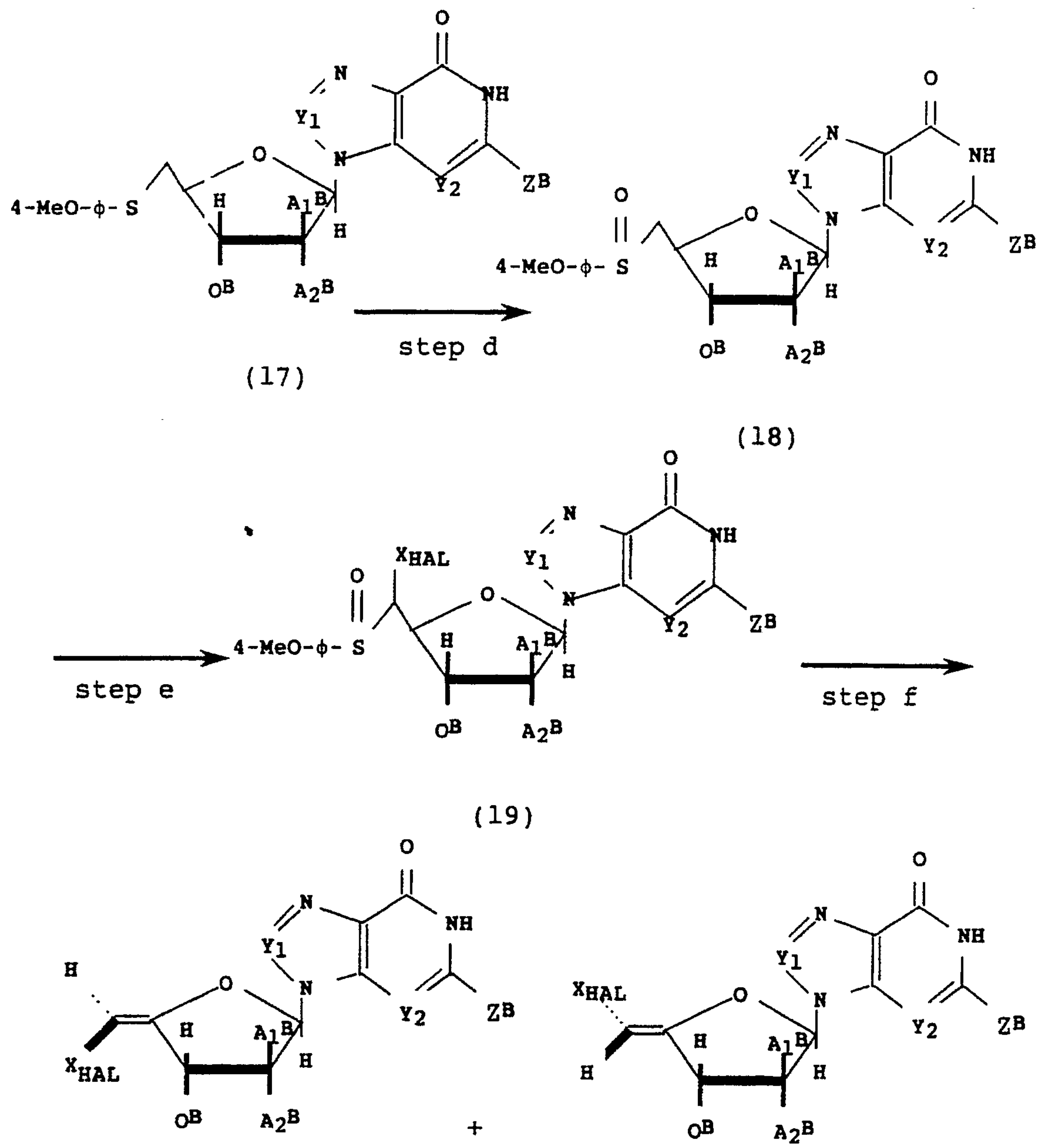


(17) (18) (19) (20) + (21)

Scheme C (cont'd)

(20) (21)

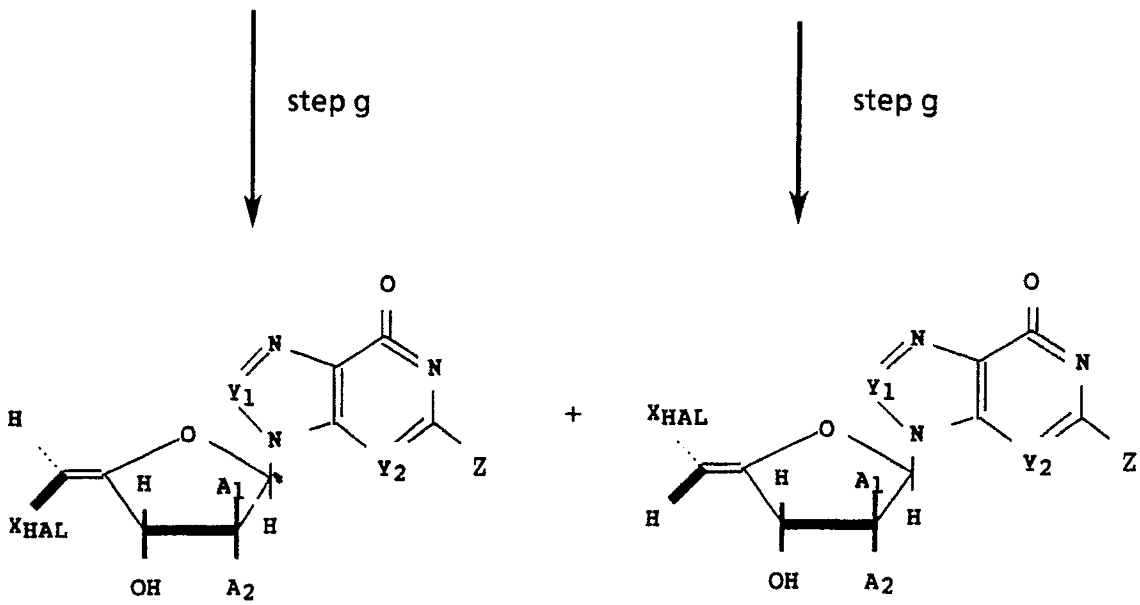


(12) (13)

In step a, reactive hydroxy groups of the appropriate inosine/guanosine derivative (14), other than the 5'-hydroxy, are blocked utilizing standard blocking agents well known in the art as described for Scheme B. Where $A_2$ is hydroxy, it is preferred to block the 2'- and 3'-hydroxy groups with a 2',3'-0-isopropylidene blocking group. Where $A_2$ is not hydroxy, it is preferred to block the 3'-hydroxy and any 2'-hydroxy (wherein $A_1$ is hydroxy) with a benzoyl group. Where a 2',3'-O-isopropylidene blocking group is not utilized, it is preferred to block the 3'-hydroxy and any 2'-hydroxy (wherein $A_1$ is hydroxy) after the reaction described in step b.

In step b, the 5'-hydroxy of the appropriately blocked 5'-hydroxy derivative (15) is subjected to a substitution reaction in which an alkyl-thio group replaces the 5'-hydroxy group to form the corresponding sulfide (16). The preferred sulfide is the 4-methoxyphenylsulfide which can be formed by reacting the appropriately blocked 5'-hydroxy derivative (15) with 4-methoxyphenyl disulfide in the presence of tributylphosphine.

In step c, any reactive amino groups, such as the 2-amino group of guanine or deoxyguanine, can be blocked as described for Scheme B. It is preferred to block the 2-amino group of guanine or deoxyguanine with an acetyl group. In addition, Where a 2',3'-O-isopropylidene blocking group is not utilized in step a, the 3'-hydroxy and any 2'-hydroxy (wherein $A_1$ is hydroxy) can be blocked as described above.

In step d, the appropriately blocked sulfide (17) is oxidized to the corresponding sulfinyl derivative (18) utilizing standard oxidizing agents well known and appreciated in the art such as 3-chloroperbenzoic acid.

In step e, the 5'-carbon of the sulfinyl derivative (18) is halogenated using a halogenating agent, such as the fluorinating agent diethylaminosulfur trifluoride (DAST), or the chlorinating agent sulfuryl chloride in the presence of a base such as pyridine, to yield the corresponding 5'-halosulfinyl derivative (19). The preferred fluorinating agent is DAST and the preferred chlorinating agent is sulfuryl chloride. Where DAST is utilized as the fluorinating agent, the fluorinated product must be re-oxidized after treatment with DAST with an equimolar amount of an oxidizing agent, such as 3-chloroperbenzoic acid, in order to provide the 5'-halo-sulfinyl derivative (19).

In step f, the sulfinyl group is then eliminated to yield the appropriately blocked 4'-vinylhalo derivatives

(20 and 21) by heating the 5′-halo-sulfinyl derivative (19) in the presence of a base such as diisopropylethyl amine.

In step g, the blocking groups of the appropriately blocked 4′-vinylhalo derivatives (20 and 21) are removed according to conventional procedures and techniques well known and appreciated in the art such as those described for Scheme B. The (Z) and (E) isomers of the 4′-vinylhaloinosine/guanosine derivative or the 4′-vinylhalodeoxyinosine/guanosine derivative, i.e., (12) and (13), are thus formed. These isomers can be separated by conventional resolution techniques well known and appreciated in the art.

Starting materials for use in the general synthetic procedure outlined in Scheme C are readily available to one of ordinary skill in the art. For example, certain starting materials for various compounds of formula (1) wherein $A_2$ is H or OH, $Y_1$ is CH, $Y_2$ is N and Z is H are commercially available. Additional starting materials can be prepared by the use of conventional methods and analogous techniques which are well known and appreciated in the art.

The following example presents a typical synthesis as described by Scheme C. This example is understood to be illustrative only and is not intended to limit the scope of the present invention in any way.

## EXAMPLE 4

### (Z)- and (E)-4′,5′-Didehydro-5′-deoxy-5′-fluoroguanosine

#### Step a: 2′,3′-O-isopropylidene-guanosine

The title compound is commercially available.

#### Step b: 5′-Deoxy-2′,3′-O-isopropylidene-5′-[(4-methoxyphenyl)thio]guanosine

To a mixture of 2′,3′-O-isopropylideneguanosine (16.1 g, 0.05 moles) and 4-methoxyphenyl disulfide (26.0 g, 0.094 moles) in dry pyridine (125 mL), add tributylphosphine (23.3 mL, 0.094 moles) via syringe. Stir under nitrogen overnight. Add water and remove solvents *in vacuo* in a rotary evaporator. Stir the residue in a mixture of water and cyclohexane. Decant the cyclohexane layer. Repeat the cyclohexane extraction two more times. Extract the water layer with ethyl acetate. Dry the ethyl acetate solution with $MgSO_4$ and concentrate to 100 mL or less. Add chloroform to the ethyl acetate solution and cool. Collect crystals of the title compound.

#### Step c: $N^2$-acetyl-5′-Deoxy-2′,3′-O-isopropylidene-5′-[ (4-methoxyphenyl) thio]guanosine

To 5′-Deoxy-2′,3′-O-isopropylidene-5′-[(4-methoxyphenyl)thio]guanosine (10 g, 0.022 moles) in 50 mL of pyridine, add 4.1 mL (2 equivalents) of acetic anhydride and stir overnight. Pour the mixture into water and extract with ethyl acetate. Dry the ethyl acetate extract with $MgSO_4$ and remove the solvents *in vacuo* to give the title compound.

Step d: $N^2$-acetyl-5′-Deoxy-2′,3′-O-isopropylidene-5′-[(4-methoxyphenyl)sulfoxyl]guanosine

To a solution of $N^2$-acetyl-5′-deoxy-2′,3′-O-isopropylidene-5′-[(4-methoxyphenyl)thio]guanosine (5 g, 0.01 moles) in 40 mL dichloromethane cooled in an ice bath, add dropwise a solution of 2.0 g (0.01 moles) of 85% 3-chloroperbenzoic acid in 40 mL dichloromethane. Stir the reaction mixture for 2 hours and partition the mixture between 400 mL chloroform and a saturated aqueous sodium bicarbonate solution. Dry the organic layer over $MgSO_4$ and evaporate the solution to dryness. Chromatograph the residue on 200 g silica gel eluting with ethyl acetate/methanol. Concentrate the eluant to yield the title compound.

#### Step e: $N^2$-acetyl-5′-Deoxy-5′-dehydro-2′,3′-O-isopropylidene-5′-fluoro-5′-[4-methoxyphenyl sulfoxyl]-quanosine

To 3.0 g of $N^2$-acetyl-5′-deoxy-2′,3′-O-isopropylidene-5′-[4-methoxyphenylsulfoxyl]guanosine (5.96

mmol) in 15 mL of 1,2-dichloroethane, add 3.15 mL (23.8 mmol) of diethylaminosulfur trifluoride. Stir the reaction mixture at 45° C for 2 hours, cool and pour into saturated aqueous sodium bicarbonate. Extract the mixture with 200 mL chloroform. Dry the extract over $MgSO_4$ and evaporate the solvents *in vacuo*. Redissolve the residue in dichloromethane and evaporate the solvents *in vacuo*. Dissolve the residue in 25 mL of dichloromethane and cool in an ice bath. To this solution, add dropwise a solution of 0.97 g of 85% 3-chloroperbenzoic acid and stir for 2 hours. Partition the reaction mixture between 200 mL chloroform and saturated aqueous sodium bicarbonate. Dry the organic layer over $MgSO_4$ and evaporate the solvents to dryness. Chromatograph the residue on 100 g silica gel eluting sequentially with cyclohexane/ethyl acetate, ethyl acetate and ethyl acetate/methanol to provide the title compound.

Step f: (Z)- and (E)-N²-acetyl-5′-Deoxy-4′,5′-didehydro-2′ ,3′-O-isopropylidene-5′-fluoroguanosine

Reflux a solution of 1.8 g of N²-acetyl-5′-deoxy-5′-dehydro-2′ ,3′-O-isopropylidene-5′-fluoro-5′-[4-methoxyphenylsulfoxyl]guanosine in 35 mL diglyme and 2.5 g diisopropylethylamine for 24 hours. Remove solvents by distillation on a Kugelrohr apparatus at 1 mm Hg. Chromatograph the residue on 50 g silica gel eluting sequentially with cyclohexane/ethyl acetate, ethyl acetate and ethyl acetate/methanol to provide a mixture of the (Z) and (E) isomers of the title compound.

Step g: (Z)- and (E)-5′-Deoxy-4′,5′-didehydro-5′-fluoroguanosine

Place 0.5 g of (Z)- and (E)-N²-acetyl-5′-deoxy-4′,5′-didehydro-2′,3′-O-isopropylidene-5′-fluoroguanosine (0.136 mmol) in 15 mL ethanol, cool to 0° C and saturate with ammonia in a pressure tube. Cap the tube and keep it at room temperature overnight. Evaporate the solvent and stir the residue in a few mL of ethyl acetate. Filter the mixture and dissolve the filter cake in 5 mL of trifluoroacetic acid/water (4/1, v/v). Stir the mixture at room temperature for 1 hour and evaporate *in vacuo*. Stir the residue in a few mL of ethyl acetate and filter to afford a 2:1 mixture of the (Z) and (E) isomers, respectively, of the title compound. Separate the isomers on a Bio-Rad® AG 1-1X resin (OH-form) under conditions described for guanosine by Dekker [*J.Am. Chem.Soc.* 87 , 4027-29 (1965)].

The following specific compounds can be made by procedures analogous to those described in Example 8:

(Z) and (E)-4′,5′-Didehydro-5′-deoxy-5′-chloro-guanosine
(Z) and (E)-4′,5′-Didehydro-5′-deoxy-5′-chloro-inosine
(Z) and (E)-4′,5′-Didehydro-2′,5′-dideoxy-5′-chloro-guanosine
(Z) and (E)-4′,5′-Didehydro-2′,5′-dideoxy-5′-chloro-inosine
(Z) and (E)-4′,5′-Didehydro-5′-deoxy-5′-fluoro-inosine
(Z) and (E)-4,,5′-Didehydro-2′,5′-dideoxy-5′-fluoro-guanosine (Z) and (E)-4′,5′-Didehydro-2′,5′-dideoxy-5′-fluoro-inosine

The 4′-vinylhalo-inosine/guanosine derivatives of the formula (1) wherein both of $X_1$ and $X_2$ are halogen, can be prepared according to the procedure described in Scheme D. This procedure can be utilized to provide compounds of formula (1) wherein $X_1$ and $X_2$ are the same halogen, as well as to provide compounds of formula (1) wherein $X_1$ and $X_2$ are different halogens. This procedure is particularly useful to provide compounds of formula (1) wherein both $X_1$ and $X_2$ are chlorine atoms and wherein one of $X_1$ and $X_2$ is a fluorine atom and the other is a chlorine atom.

Scheme D

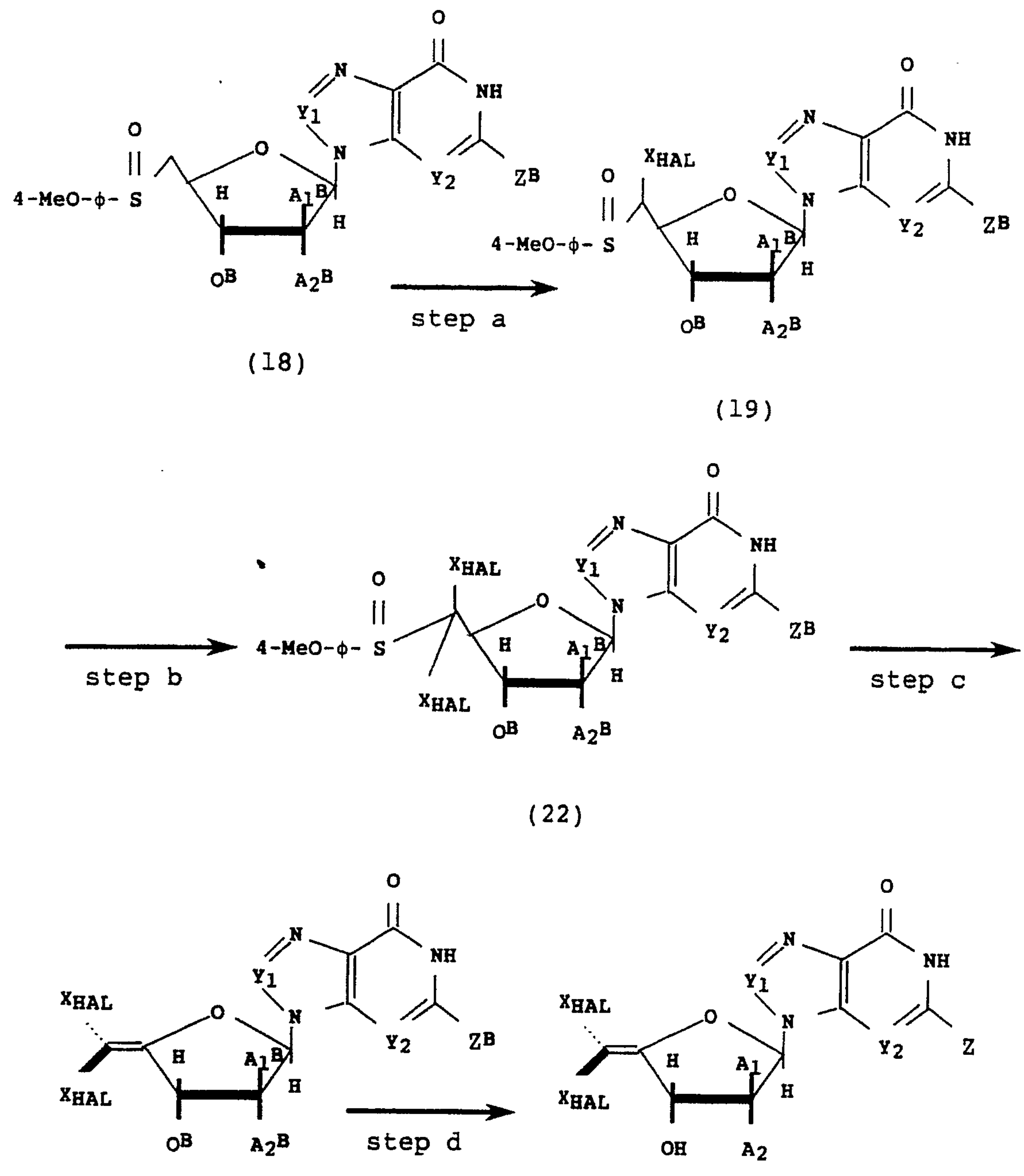


(18) (19) (22) (23) (24)

In step a, the 5′-carbon of the sulfinyl derivative (18), which is prepared as described in Scheme C, is halogenated using a halogenating agent to yield the corresponding 5′-halo-sulfinyl derivative (19). Where fluorination is desired, it is preferred to treat the sulfinyl derivative (18) with the fluorinating agent DAST followed by reoxidation with 3-chloroperbenzoic acid as described for Scheme C. Where chlorination is desired, it is preferred to treat the sulfinyl derivative (18) with the chlorinating agent sulfuryl chloride in the presence of a base such as pyridine.

In step b, halogenation of the 5′-carbon of the 5′-halosulfinyl derivative (19) is continued so as to provide a 5′,5′-dihalo-sulfinyl derivative (22). Where compounds of formula (1) are desired wherein $X_1$ and $X_2$ are both chlorine atoms, the sulfinyl derivative (18), depicted in step a, is treated with 2 equivalents of sulfuryl chloride to yield the corresponding 5′,5′-dichloro-sulfinyl derivative. Where compounds of formula (1) are desired wherein $X_1$ and $X_2$ are different halogens, the sulfinyl derivative (18) is treated with 1

equivalent of the appropriate halogenating agent. The 5′-halo-sulfinyl derivative (19) is isolated and then treated with 1 equivalent of the appropriate different halogenating agent.

For example, where compounds of formula (1) are desired wherein one of $X_1$ and $X_2$ is a fluorine atom and the other is a chlorine atom, the sulfinyl derivative (18) is treated with 1 equivalent of a fluorinating agent such as DAST, and reoxidized as described above, to yield the corresponding 5′-fluoro-sulfinyl derivative. The 5′-fluoro-sulfinyl derivative is then chlorinated with 1 equivalent of a chlorinating agent such as sulfuryl chloride to yield the corresponding 5′-fluoro-5′-chloro-sulfinyl derivative. For example, the 5′-fluoro-sulfinyl derivative (19) can be reacted with sulfuryl chloride in dichloromethane in the presence of pyridine.

In step c, the sulfinyl group of the 5′,5′-dihalosulfinyl derivative (22) is eliminated as described in Scheme C, step f, to yield the appropriately blocked 4′-vinyl-dihalo derivative (23).

In step d, the blocking groups of the appropriately blocked 4′-vinyl-dihalo derivative (23) are removed as described in Scheme C, step g, to yield the 4′-vinyl-dihalo derivative (24).

It is of course understood that where $X_1$ and $X_2$ are each different halogens, the 4′-vinyl-dihalo derivative (24) will exist as a mixture of the (Z) and (E) isomers. These isomers can be readily separated by conventional techniques and procedures as are well known and appreciated in the art.

The following example presents a typical synthesis as described by Scheme C. This example is understood to be illustrative only and is not intended to limit the scope of the present invention in any way.

## EXAMPLE 5

### (Z)- and (E)-4′,5′-Didehydro-5′-deoxy-5′-fluoro-5′-chloro-guanosine

#### Step a: $N^2$-acetyl-5′-Deoxy-2′,3′-O-isopropylidene-5′-fluoro-5′-[4-methoxyphenylsulfoxyl]guanosine

To 3.0 g of $N^2$-acetyl-5′-deoxy-2′,3′-O-isopropylidene-5′-[4-methoxyphenylsulfoxyl]guanosine (5.96 mmol) in 15 mL of 1,2-dichloroethane, add 3.15 mL (23.8 mmol) of diethylaminosulfur trifluoride. Stir the reaction mixture at 45°C for 2 hours, cool and pour into saturated aqueous sodium bicarbonate. Extract the mixture with 200 mL chloroform. Dry the extract over $MgSO_4$ and evaporate the solvents *in vacuo*. Redissolve the residue in dichloromethane and evaporate the solvents *in vacuo*. Dissolve the residue in 25 mL of dichloromethane and cool in an ice bath. To this solution, add dropwise a solution of 0.97 g of 85% 3-chloroperbenzoic acid and stir for 2 hours. Partition the reaction mixture between 200 mL chloroform and saturated aqueous sodium bicarbonate. Dry the organic layer over $MgSO_4$ and evaporate the solvents to dryness. Chromatograph the residue on 100 g silica gel eluting sequentially with cyclohexane/ethyl acetate, ethyl acetate and ethyl acetate/methanol to provide the title compound.

#### Step b: $N^2$-acetyl-5′-Deoxy-2′,3′-O-isopropylidene-5′-fluoro-5′-chloro-5′-[4-methoxyphenylsulfoxyl]guanosine

To $N^2$-acetyl-5′-deoxy-2′,3′-O-isopropylidene-5′-fluoro-5′-[4-methoxyphenylsulfoxyl]guanosine (0.89 g, 1.7 mmol) in 6.5 mL of dry dichloromethane add 0.32 mL (4.0 mmol) of pyridine and cool the mixture in an ice bath under nitrogen. To this mixture, add 0.18 mL (1.9 mmol) of $SO_2Cl_2$ (sulfuryl chloride) and stir the mixture for 20 minutes. Remove the solvents *in vacuo* to afford a foam. Percolate the product through a silica gel column eluting with dichloromethane followed by ethyl acetate/dichloromethane (1:4, v/v). Repeat the percolation procedure to yield the title compound as a foam. Triturate the product with dichloromethane/cyclohexane to yield a solid.

#### Step c: (Z)- and (E)-$N^2$-acetyl-5′-Deoxy-4′,5′-didehydro-2′,3′-O-isopropylidene-5′-fluoro-5′-chloro-guanosine

Reflux a solution of 1.0 g of $N^2$-acetyl-5′-deoxy-2′,3′-O-isopropylidene-5′-fluoro-5′-chloro-5′-[4-methoxyphenylsulfoxyl]guanosine in 35 mL diglyme and 2.5 g diisopropylethylamine for 24 hours. Remove solvents by distillation on a Kugelrohr apparatus at 1 mm Hg. Chromatograph the residue on 50 g silica gel eluting sequentially with cyclohexane/ethyl acetate, ethyl acetate and ethyl acetate/methanol to provide a

mixture of the (Z) and (E) isomers of the title compound.

Step d: (Z)- and (E)-5′-Deoxy-4′,5′-didehydro-5′-fluoro-5′-chloro-guanosine

Place 0.4 g of (Z)- and (E)-$N^2$-acetyl-5′-deoxy-4′,5′-didehydro-2′ ,3′ -O-isopropylidene-5′-fluoro-5′-chloro-guanosine (1.0 mmol) in 15 mL ethanol, cool to 0° C and saturate with ammonia in a pressure tube. Cap the tube and keep it at room temperature overnight. Evaporate the solvent and stir the residue in a few mL of ethyl acetate. Filter the mixture and dissolve the filter cake in 5 mL of trifluoroacetic acid/water (4/1, v/v). Stir the mixture at room temperature for 1 hour and evaporate *in vacuo*. Stir the residue in a few mL of ethyl acetate and filter to afford a mixture of the (Z) and (E) isomers of the title compound. Separate the isomers on a Bio-Rad® AG 1-1X resin (OH-form) under conditions described for guanosine by Dekker [*J.Am. Chem.Soc.* 87 , 4027-29 (1965)].

The following specific compounds can be made by procedures analogous to those described in Example 8:

(Z)- and (E)-5′-Deoxy-4′,5′-didehydro-5′-fluoro-5′-chloro-inosine
(Z)- and (E)-2′,5′-Dideoxy-4′,5′-didehydro-5′-fluoro-5′-chloro-inosine
(Z)- and (E)-2′,5′-Dideoxy-4′,5′-didehydro-5′-fluoro-5′-chloro-guanosine
(Z)- and (E)-5′-Deoxy-4′,5′-didehydro-5′,5′-dichloro-inosine
(Z)- and (E)-2′,5′-Dideoxy-4′,5′-didehydro-5′,5′-dichloro-inosine
(Z)- and (E)-5′-Deoxy4′,5′-didehydro-5′,5′-dichloro-guanosine
(Z)- and (E)-2′,5′-Dideoxy-4′,5′-didehydro-5′,5′-dichloro-guanosine.

The present invention provides a method of effecting immunosuppression in a patient in need thereof by administering to said patient a therapeutically effective immunosuppressive amount of a compound of formula (1). By administering a compound of formula (1) to a patient, an immunosuppressive effect is provided. It is believed that this immunosuppressive effect is generated through an inhibition of purine nucleoside phosphorylase, however this invention is not limited by the proposed mechanism by which it occurs.

According to the present invention, the term "immunosuppressive effect" refers to an effect provided by immunosuppressive agents wherein the cellular immunity of the patient is substantially suppressed or inhibited. This suppression of cellular immunity can occur by inhibition of lymphocyte proliferation and, more particularly, by inhibition of T-lymphocyte proliferation. It is understood that the term "immunosuppressive effect" does not necessarily refer to complete suppression or inhibition but refers to at least a substantial suppression or inhibition of the cellular immunity or lymphocyte proliferation of the patient.

As used herein, the term "patient" refers to a warm-blooded animal such as a mammal which is afflicted with a disease or condition wherein the patient is in need of treatment with an immunosuppressive agent in order to provide an immunosuppressive effect. It is understood that humans are included within the scope of the meaning of the term.

A patient can be in need of treatment with an immunosuppressive agent where the patient is suffering from a chronic allergic reaction which produces unwanted and troublesome cell-mediated allergic symptoms. These symptoms can be alleviated through suppression or inhibition of cell-mediated immune response.

A patient can also be in need of treatment with an immunosuppressive agent where the patient is suffering from an autoimmune disease including various collagen-vascular disorders such as rheumatoid arthritis, systemic lupus erythematosis (SLE), necrotizing vasculitus, scleroderma, polymyositus, and related entities such as Wegener's granulomatosis, as well as regional enteritis, ulcerative colitis, chronic active hepatitis, glumerulonephritus, the nephrotic syndrome, Goodpasture's syndrome, autoimmune hemolytic anemia, idiopathic thrombocytopenic purpura, pemphigus, and the like. In these autoimmune diseases, the patient's own cellular immune system attacks its own tissues. Symptoms of these autoimmune diseases can be alleviated through suppression or inhibition of cell-mediated immune response.

A patient can also be in need of treatment with an immunosuppressive agent where the patient is in danger of rejection of a transplanted tissue or organ. Where patients receive a homograft which is not histocompatible with their own tissues, the patient's immune system can reject the homograft through a cell-mediated immune response. In these cases, suppression or inhibition of the cell-mediated immune response can prevent the rejection of the tissue or organ and make transplantation across histocompatibility barriers feasible.

Based on standard clinical and laboratory tests and procedures, an attending diagnostician, as a person

skilled in the art, can readily identify those patients who are in need of treatment with immunosuppressive agents.

In effecting treatment of a patient, a compound of formula (1) can be administered in any form or mode which makes the compound bioavailable in therapeutically effective immunosuppressive amounts, including oral and parenteral routes. For example, compounds of formula (1) can be administered orally, topically, subcutaneously, intramuscularly, intravenously, transdermally, intranasally, rectally, and the like. Oral administration is generally preferred. One skilled in the art can readily select the proper form and mode of administration depending upon the particular characteristics of the compound selected the disease state to be treated, the stage of the disease, and other relevant circumstances.

As used herein, the term "therapeutically effective immunosuppressive amount" refers to an amount of the compound of formula (1) which is effective in suppressing or inhibiting a cell-mediated immune response. This term also refers to that amount which is effective in inhibiting lymphocyte proliferation. A therapeutically effective immunosuppressive amount can be readily determined by the attending diagnostician, as one skilled in the art, by the use of known techniques and by observing results obtained under analogous circumstances. In determining the therapeutically effective immunosuppressive amount, a number of factors are considered by the attending diagnostician, including, but not limited to: the species of mammal; its size, age, and general health; the specific disease involved; the degree of or involvement or the severity of the disease; the response of the individual patient; the particular compound administered; the mode of administration; the bioavailability characteristics of the preparation administered; the dose regimen selected; the use of concomitant medication; and other relevant circumstances.

A therapeutically effective immunosuppressive amount of a compound of formula (1) is expected to vary from about 0.1 milligram per kilogram of body weight per day (mg/kg/day) to about 100 mg/kg/day. Preferred amounts are expected to vary from about 1 to about 20 mg/kg/day. These ranges are particularly reflective of effective amounts upon oral administration but also are reflective of the operable ranges for parenteral administration.

The compounds can be administered alone or in the form of a pharmaceutical composition in combination with pharmaceutically acceptable carriers or excipients, the proportion and nature of which are determined by the solubility and chemical properties of the compound selected, the chosen route of administration, and standard pharmaceutical practice. The compounds of the invention, while effective themselves, may be formulated and administered in the form of their pharmaceutically acceptable acid addition salts for purposes of stability, convenience of crystallization, increased solubility and the like.

The compounds of formula (1) may be provided as compositions comprising an assayable amount of a compound of formula (1) in admixture with one or more inert carriers.

These compositions are useful, for example, as assay standards, as convenient means of making bulk shipments, or as pharmaceutical compositions. An assayable amount of a compound of formula (1) is an amount which is readily measurable by standard assay procedures and techniques as are well known and appreciated by those skilled in the art. Assayable amounts of a compound of formula (1) will generally vary from about 0.001% to about 75% of the composition by weight. Inert carriers can be any material which does not degrade or otherwise covalently react with a compound of formula (1). Examples of suitable inert carriers are water; aqueous buffers, such as those which are generally useful in High Performance Liquid Chromatography (HPLC) analysis; organic solvents, such as acetonitrile, ethyl acetate, hexane and the like; and pharmaceutically acceptable carriers or excipients. These compositions are prepared by mixing the compound of formula (1) with the inert carriers utilizing techniques and methods which are well known and appreciated in the art.

More particularly, the compounds of formula (1) may be provided in pharmaceutical compositions comprising a therapeutically effective antiprotozoal amount of a compound of formula (1) in admixture or otherwise in association with one or more pharmaceutically acceptable carriers or excipients.

The pharmaceutical compositions are prepared in a manner well known in the pharmaceutical art. The carrier or excipient may be a solid, semi-solid, or liquid material which can serve as a vehicle or medium for the active ingredient. Suitable carriers or excipients are well known in the art. The pharmaceutical composition may be adapted for oral or parenteral use and may be administered to the patient in the form of tablets, capsules, suppositories, solution, suspensions, or the like.

The compounds of formula (1) may be administered orally, for example, with an inert diluent or with an edible carrier. They may be enclosed in gelatin capsules or compressed into tablets. For the purpose of oral therapeutic administration, the compounds may be incorporated with excipients and used in the form of tablets, troches, capsules, elixirs, suspensions, syrups, wafers, chewing gums and the like. These preparations should contain at least 4% of the compound of the invention, the active ingredient, but may be varied depending upon the particular form and may conveniently be between 4% to about 70% of the weight of

the unit. The amount of the compound present in compositions is such that a suitable dosage will be obtained. Preferred compositions and preparations according to the present invention are prepared so that an oral dosage unit form contains between 5.0-300 milligrams of a compound of the invention.

The tablets, pills, capsules, troches and the like may also contain one or more of the following adjuvants: binders such as microcrystalline cellulose, gum tragacanth or gelatin; excipients such as starch or lactose, disintegrating agents such as alginic acid, Primogel, corn starch and the like; lubricants such as magnesium stearate or Sterotex; glidants such as colloidal silicon dioxide; and sweetening agents such as sucrose or saccharin may be added or a flavoring agent such as peppermint, methyl salicylate or orange flavoring. When the dosage unit form is a capsule, it may contain, in addition to materials of the above type, a liquid carrier such as polyethylene glycol or a fatty oil. Other dosage unit forms may contain other various materials which modify the physical form of the dosage unit, for example, as coatings. Thus, tablets or pills may be coated with sugar, shellac, or other enteric coating agents. A syrup may contain, in addition to the present compounds, sucrose as a sweetening agent and certain preservatives, dyes and colorings and flavors. Materials used in preparing these various compositions should be pharmaceutically pure and non-toxic in the amounts used.

For the purpose of parenteral therapeutic administration, the compounds of formula (1) may be incorporated into a solution or suspension. These preparations should contain at least 0.1% of a compound of the invention, but may be varied to be between 0.1 and about 50% of the weight thereof. The amount of the compound of formula (1) present in such compositions is such that a suitable dosage will be obtained. Preferred compositions and preparations according to the present invention are prepared so that a parenteral dosage unit contains between 5.0 to 100 milligrams of the compound of the invention.

The solutions or suspensions may also include the one or more of the following adjuvants: sterile diluents such as water for injection, saline solution, fixed oils, polyethylene glycols, glycerine, propylene glycol or other synthetic solvents; antibacterial agents such as benzyl alcohol or methyl paraben; antioxidants such as ascorbic acid or sodium bisulfite; chelating agents such as ethylene diaminetetraacetic acid; buffers such as acetates, citrates or phosphates and agents for the adjustment of tonicity such as sodium chloride or dextrose. The parenteral preparation can be enclosed in ampules, disposable syringes or multiple dose vials made of glass or plastic.

In addition, the present invention also provides a method of inhibiting a purine nucleoside phosphorylase in a patient in need thereof comprising administering to said patient an effective purine nucleoside phosphorylase inhibitory amount of a compound of formula (1). By administering an effective purine nucleoside phosphorylase inhibitory amount of a compound of formula (1), the purine nucleoside phosphorylase of the patient is inhibited, thereby providing an immunosuppressive effect. Compounds of formula (1) are administered as described above. The term "effective purine nucleoside phosphorylase inhibitory amount" refers to that amount of a compound of formula (1) which is effective in substantially inhibiting the purine nucleoside phosphorylase in the patient so as to provide an immunosuppressive effect. An effective purine nucleoside phosphorylase inhibitory amount is the same as a therapeutically effective immunosuppressive amount as described above.

As with any group of structurally related compounds which possesses a particular generic utility, certain groups and configurations are preferred for compounds of formula (1) in their end-use application..

With respect to the substituents $X_1$ and $X_2$, compounds of formula (1) wherein $X_1$ is fluoro and $X_2$ is hydrogen, and those wherein $X_1$ is hydrogen and $X_2$ is fluoro, are generally preferred. With respect to the substituent $A_1$, compounds of formula (1) wherein $A_1$ is hydrogen are generally preferred. With respect to the substituent $A_2$, compounds of formula (1) wherein $A_2$ is hydroxy are generally preferred. Furthermore, compounds of formula (1) wherein $Y_1$ is a CH group and $Y_2$ is nitrogen are generally preferred.

## Claims

1. A compound of the formula 1

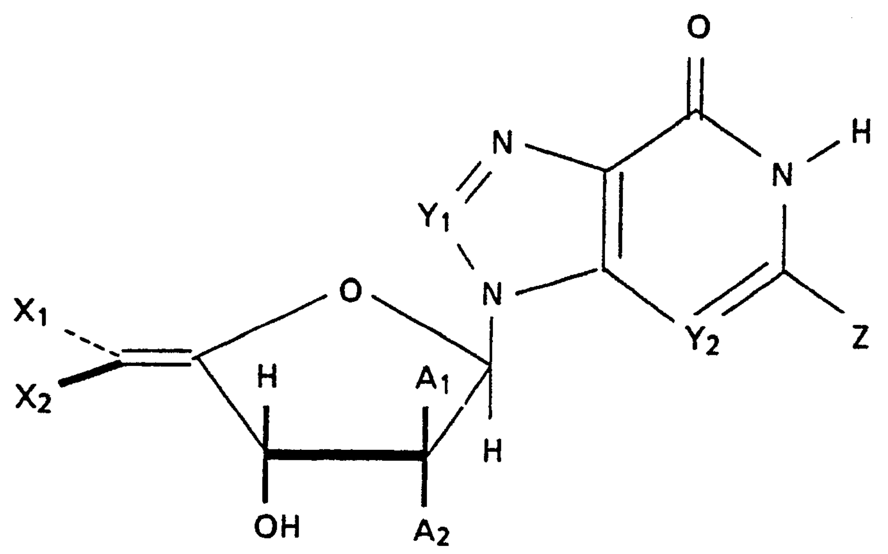

(1)

wherein
$X_1$ and $X_2$ are each independently hydrogen or halogen with the proviso that at least one of $X_1$ and $X_2$ is a halogen atom,
$A_1$ and $A_2$ are each independently hydrogen, halogen or hydroxy with the provisos that where $A_1$ is hydroxy, $A_2$ is hydrogen, and where $A_2$ is hydroxy, $A_1$ is hydrogen,
$Y_1$ is nitrogen, a CH group, a CCl group, a CBr group or a $CNH_2$ group,
$Y_2$ is nitrogen or a CH group, and
Z is hydrogen, halogen, or $NH_2$.

2. A process for preparing a compound of the formula 3

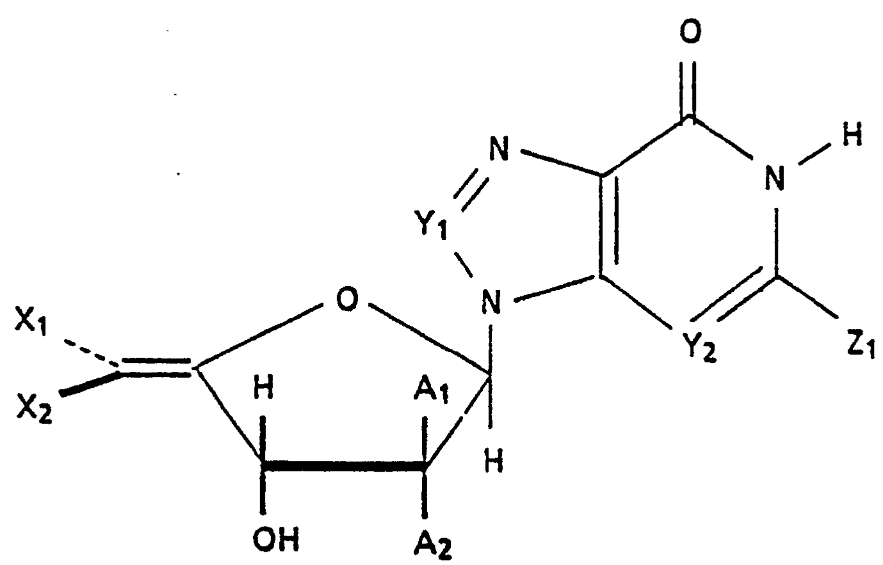

(3)

wherein $X_1$, $X_2$, $A_1$, $A_2$, $Y_1$ and $Y_2$ have the same meaning as in claim 1, and $Z_1$ is hydrogen or halogen, comprising reacting an acidic oxidative agent with a compound of the formula 2

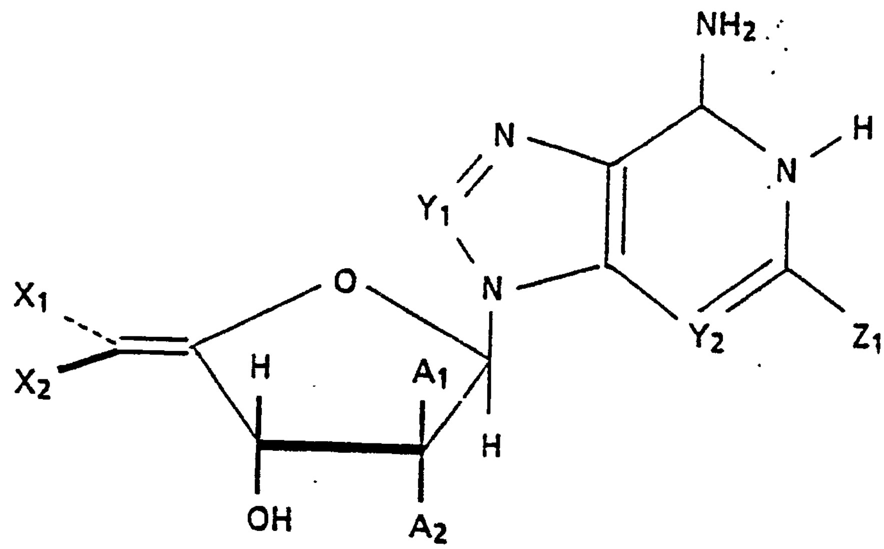

(2)

wherein the substituents $X_1$, $X_2$, $A_1$, $A_2$, $Y_1$, $Y_2$ and $Z_1$ have the above meaning.

3. A process for preparing a compound of the formula 1

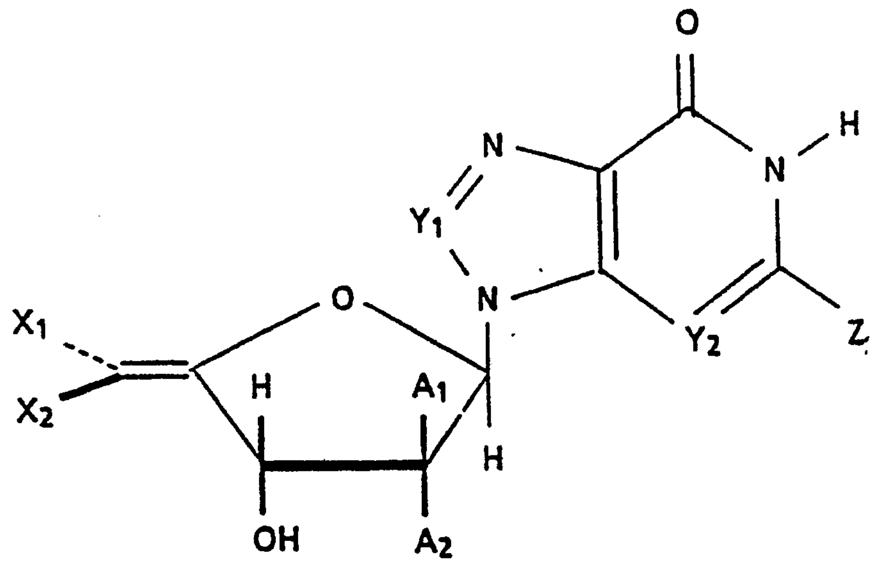

(1)

wherein $X_1$, $X_2$, $A_1$, $A_2$, $Y_1$, $Y_2$ and Z have the same meaning as in claim 1 comprising reacting a compound of the formula

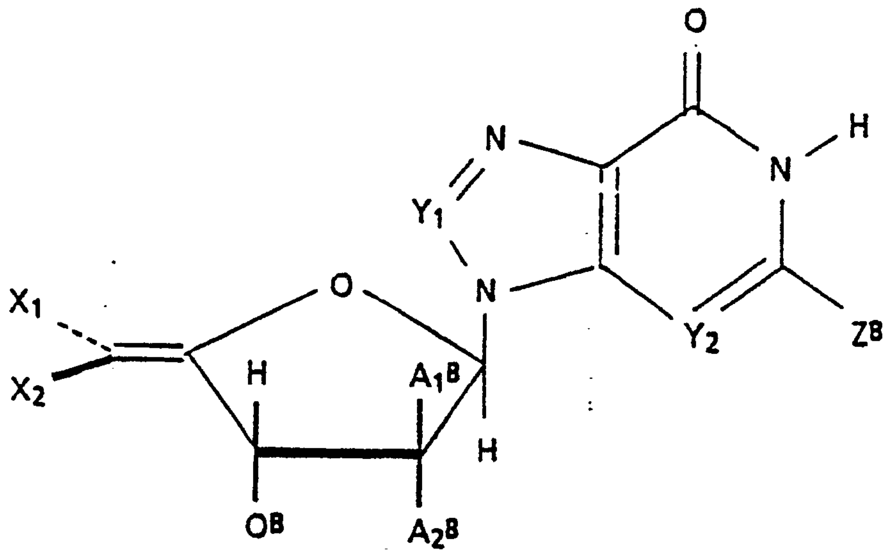

wherein $X_1$, $X_2$, $Y_1$ and $Y_2$ have the above meaning and $O^B$, $A_1{}^B$ and $A_2{}^B$ represent OH, $A_1$ and $A_2$ groups, respectively, bearing hydroxy-blocking groups where appropriate, and $Z^B$ represents a Z group bearing an amino-blocking group where appropriate,

(a) with an acid to remove any hydroxy-protecting groups and

(b) with a base to remove any amino-protecting group.

4. Use of a compound of claim 1 for the preparation of a pharmaceutical composition.

5. Use of a compound of claim 1 for the preparation of a pharmaceutical composition which is suited for effecting immunosuppression in a patient.

6. The use according to claim 5 wherein the pharmaceutical composition is suited for suppressing cellular immunity in a patient in need thereof.

7. Use of a compound of claim 1 for the preparation of a pharmaceutical composition for inhibiting lymphocyte proliferation in a patient in need thereof.

8. Use according to any of claims 5, 6, or 7 wherein the patient is suffering from an autoimmune disease.

9. Use according to any of claims 5, 6, or 7 wherein the patient is in danger of suffering from rejection of a transplanted tissue or organ.

10. Use according to any of claims 5, 6, or 7 wherein the patient is suffering from a chronic allergic reaction.

11. A pharmaceutical composition comprising a therapeutically effective amount of a compound of claim 1 in admixture with one or more pharmaceutically acceptable carriers or excipients.

12. A pharmaceutical composition according to claim 11 exhibiting immunosuppressive effectivity.

Claims for the following Contracting State:GR

1. A compound of the formula 1

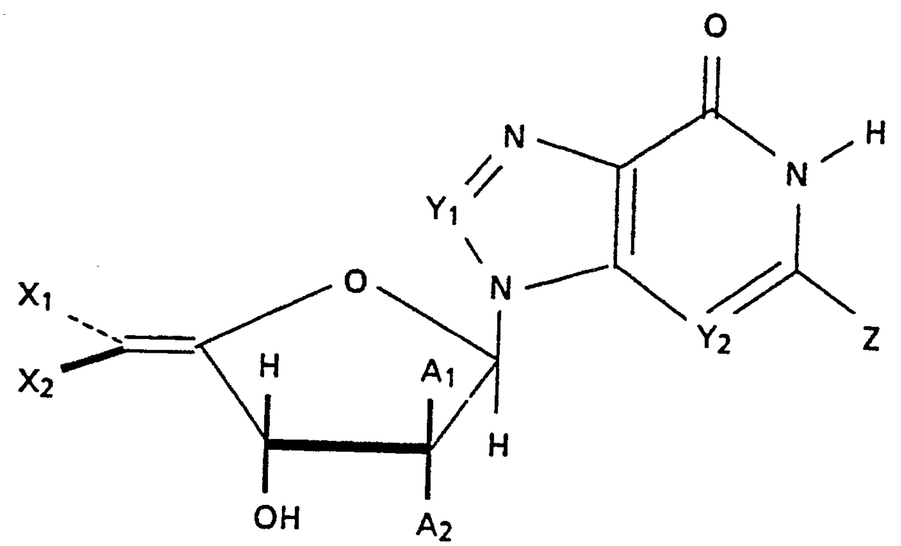

(1)

wherein

$X_1$ and $X_2$ are each independently hydrogen or halogen with the proviso that at least one of $X_1$ and $X_2$ is a halogen atom,

$A_1$ and $A_2$ are each independently hydrogen, halogen or hydroxy with the provisos that where $A_1$ is hydroxy, $A_2$ is hydrogen, and where $A_2$ is hydroxy, $A_1$ is hydrogen,

$Y_1$ is nitrogen, a CH group, a CCl group, a CBr group or a $CNH_2$ group,

$Y_2$ is nitrogen or a CH group, and

Z is hydrogen, halogen, or $NH_2$.

2. A process for preparing a compound of the formula 3

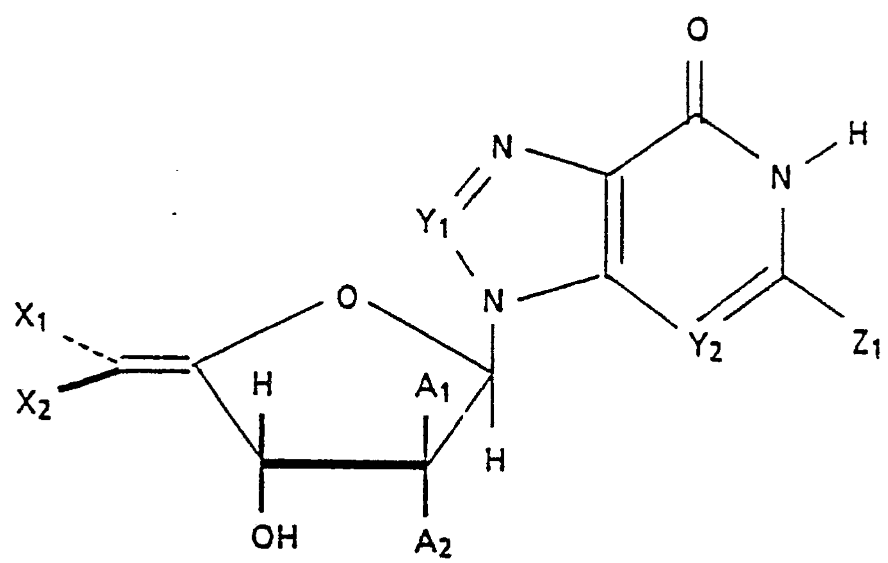

(3)

wherein $X_1$, $X_2$, $A_1$, $A_2$, $Y_1$ and $Y_2$ have the same meaning as in claim 1, and $Z_1$ is hydrogen or halogen, comprising reacting an acidic oxidative agent with a compound of the formula 2

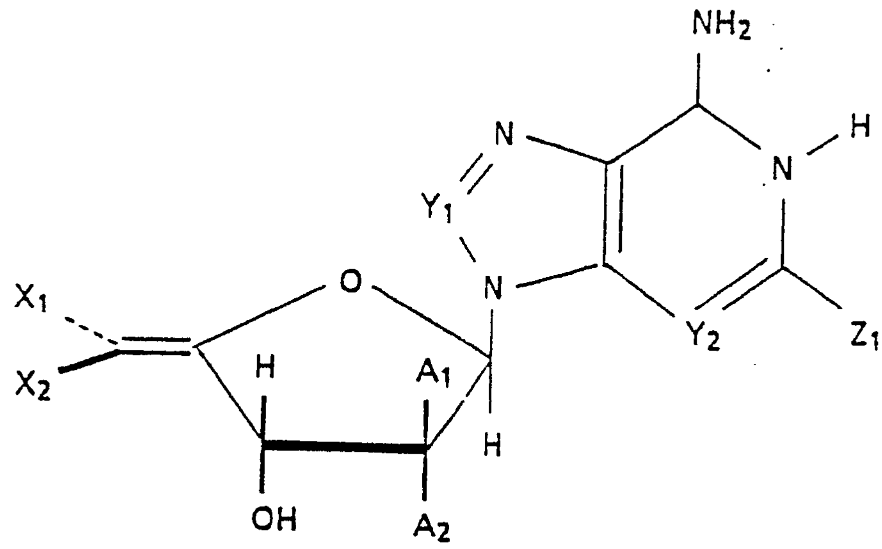

(2)

wherein the substituents $X_1$, $X_2$, $A_1$, $A_2$, $Y_1$, $Y_2$ and $Z_1$ have the above meaning.

3. A process for preparing a compound of the formula 1

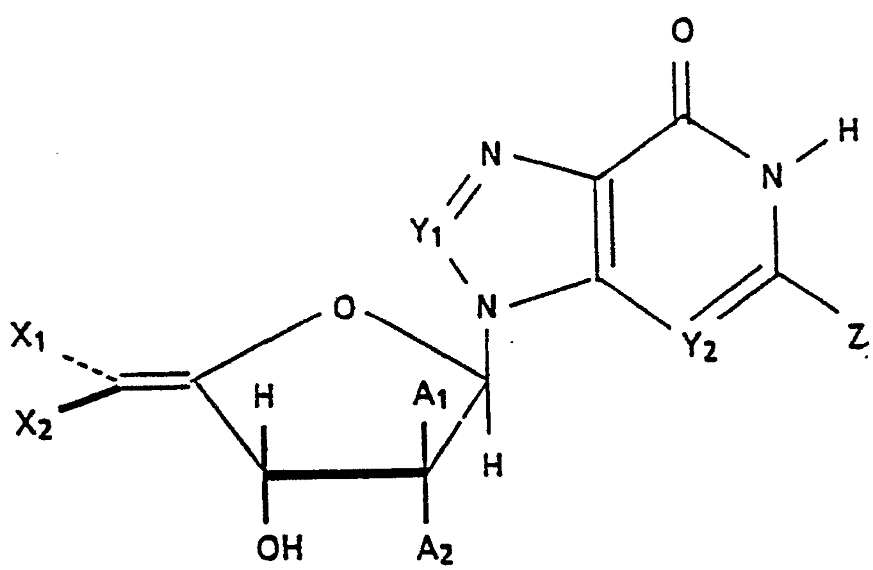

(1)

wherein $X_1$, $X_2$, $A_1$, $A_2$, $Y_1$, $Y_2$ and Z have the same meaning as in claim 1 comprising reacting a compound of the formula

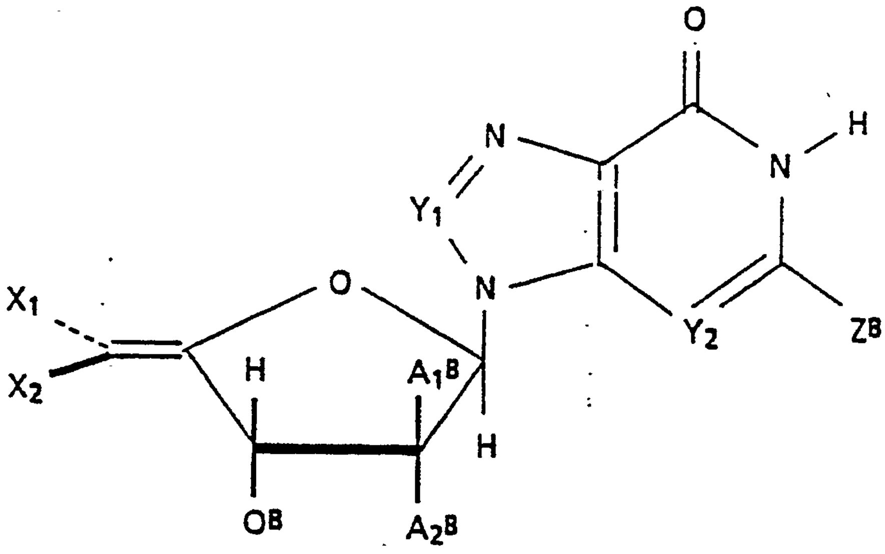

wherein $X_1$, $X_2$, $Y_1$ and $Y_2$ have the above meaning and $O^B$, $A_1{}^B$ and $A_2{}^B$ represent OH, $A_1$ and $A_2$ groups, respectively, bearing hydroxy-blocking groups where appropriate, and $Z^B$ represents a Z group bearing an amino-blocking group where appropriate,

(a with an acid to remove any hydroxy-protecting groups and

(b) with a base to remove any amino-protecting group.

4. Use of a compound of claim 1 for the preparation of a pharmaceutical composition.

5. Use of a compound of claim 1 for the preparation of a pharmaceutical composition which is suited for effecting immunosuppression in a patient.

6. The use according to claim 5 wherein the pharmaceutical composition is suited for suppressing cellular immunity in a patient in need thereof.

7. Use of a compound of claim 1 for the preparation of a pharmaceutical composition for inhibiting lymphocyte proliferation in a patient in need thereof.

8. Use according to any of claims 5, 6, or 7 wherein the patient is suffering from an autoimmune disease.

9. Use according to any of claims 5, 6, or 7 wherein the patient is in danger of suffering from rejection of a transplanted tissue or organ.

10. Use according to any of claims 5, 6, or 7 wherein the patient is suffering from a chronic allergic reaction.

Claim for the following Contracting State: ES

1. A process for preparing a compound of the formula 1

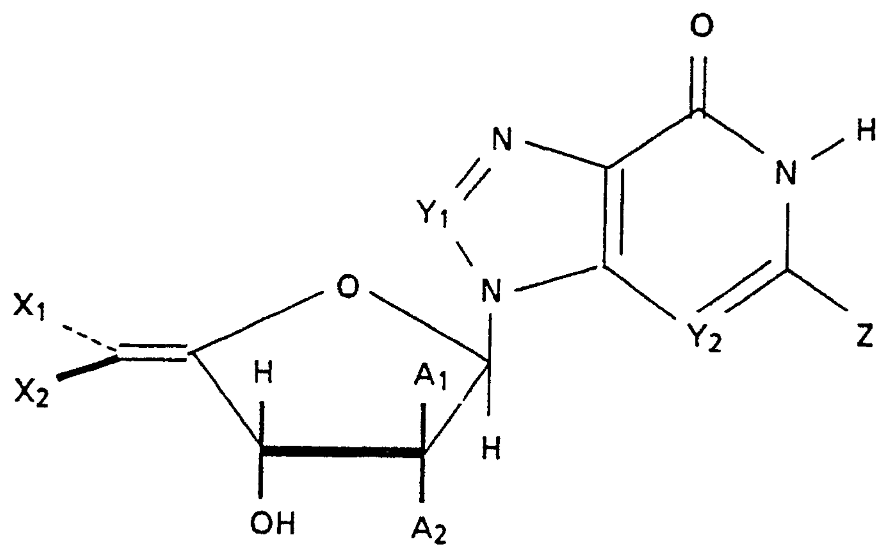

(1)

wherein

$X_1$ and $X_2$ are each independently hydrogen or halogen with the proviso that at least one of $X_1$ and $X_2$ is a halogen atom,

$A_1$ and $A_2$ are each independently hydrogen, halogen or hydroxy with the provisos that where $A_1$ is hydroxy, $A_2$ is hydrogen, and where $A_2$ is hydroxy, $A_1$ is hydrogen,

$Y_1$ is nitrogen, a CH group, a CCl group, a CBr group or a $CNH_2$ group,

$Y_2$ is nitrogen or a CH group, and

Z is hydrogen, halogen, or $NH_2$

comprising

1.1 when preparing compound 1 wherein Z is other than $NH_2$ reacting an oxidative agent with a compound of the formula 2

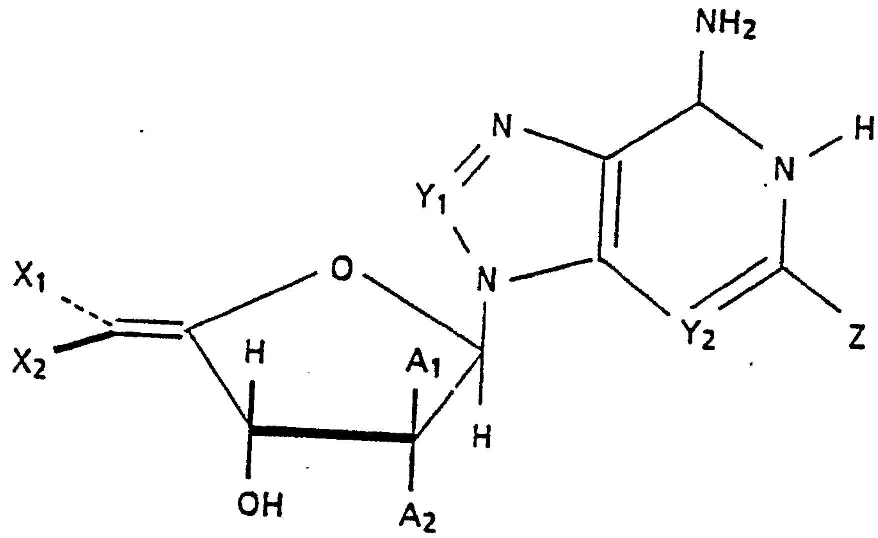

(2)

wherein the substituents $X_1$, $X_2$, $A_1$, $A_2$, $Y_1$, $Y_2$ and Z have the above meaning; and

1.2 when preparing compound 1 wherein Z is hydrogen, halogen or $NH_2$ reacting a compound of the formula

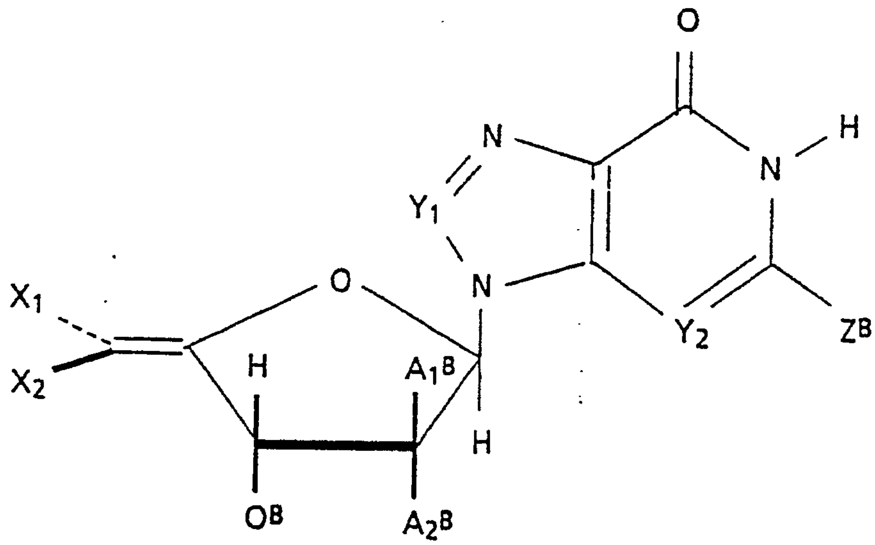

wherein $O^B$, $A_1{}^B$ and $A_2{}^B$ represent OH, $A_1$ and $A_2$ groups, respectively, bearing hydroxy-blocking groups where appropriate, and $Z^B$ represents a Z group bearing an amino-blocking group where appropriate,

(a) with an acid to remove any hydroxy-protecting groups and

(b) with a base to remove any amino-protecting group.

European Patent Office

# EUROPEAN SEARCH REPORT

Application Number

**EP 90 11 9495**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | EP-A-0 334 561 (MERRELL DOW PHARMACEUTICALS INC.)<br>* Abstract; pages 2,3 * | 1 | C 07 H 19/052<br>C 07 H 19/056<br>C 07 H 19/167<br>C 07 H 19/173<br>C 07 H 19/19 |
| A | EP-A-0 304 889 (MERRELL DOW PHARMACEUTICALS INC.)<br>* Abstract; page 2, line 1 - page 3, line 40 * | 1 | |
| A | THE JOURNAL OF ORGANIC CHEMISTRY, vol. 44, no. 3, 1979, pages 400-456, The American Chemical Society; S.D. DIMITRIJEVICH et al.: "Halo sugar nucleosides. 6. Synthesis of some 5'-deoxy-5'-iodo and 4',5'-unsaturated purine nucleosides"<br>* Abstract; page 401, compound 8; page 402, compound 22 * | 1 | |
| A | WO-A-8 703 617 (SCRIPPS CLINIC AND RESEARCH FOUNDATION)<br>* Claim 1 * | 1,4-6 | |
| | | | **TECHNICAL FIELDS SEARCHED (Int. Cl.5)** |
| | | | C 07 H 19/00<br>A 61 K 31/00 |

The present search report has been drawn up for all claims

| Place of search | Date of completion of search | Examiner |
|---|---|---|
| The Hague | 25 January 91 | SCOTT J.R.M. |

CATEGORY OF CITED DOCUMENTS

X: particularly relevant if taken alone
Y: particularly relevant if combined with another document of the same catagory
A: technological background
O: non-written disclosure
P: intermediate document
T: theory or principle underlying the invention

E: earlier patent document, but published on, or after the filing date
D: document cited in the application
L: document cited for other reasons

&: member of the same patent family, corresponding document